(19) Europäisches Patentamt
European Patent Office
Office européen des brevets

(11) EP 1 256 628 A2

(12) EUROPEAN PATENT APPLICATION

(43) Date of publication:
13.11.2002 Bulletin 2002/46

(51) Int Cl.[7]: **C12N 15/54**, C12N 9/12, C07K 14/18, C12Q 1/25

(21) Application number: **02009387.8**

(22) Date of filing: **07.05.2002**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **10.05.2001 US 289829 P**

(71) Applicant: **Agouron Pharmaceuticals, Inc.**
**La Jolla, CA 92037 (US)**

(72) Inventors:
- **Love, Robert A.**
  **San Diego, California 92129 (US)**
- **Yu, Xiu**
  **San Diego CA 92130 (US)**
- **Diehl, Wade**
  **San Diego CA 92101 (US)**
- **Hickey, Michael John**
  **San Diego CA 92126 (US)**
- **Parge, Hans E.**
  **San Diego CA 92103 (US)**
- **Gao, Jingjin**
  **San Diego CA 92128 (US)**
- **Fuhrman Shella**
  **San Diego CA 92110 (US)**

(74) Representative: **Keller, Günter, Dr.**
**Lederer & Keller**
**Patentanwälte**
**Prinzregentenstrasse 16**
**80538 München (DE)**

(54) **Hepatitis c virus (hcv) ns5b rna polymerase and mutants thereof**

(57) The present invention generally relates to viral RNA polymerases, which are essential to viral reproduction and propagation in a host. Viral RNA polymerases are known to play a role in the replication of the virus once it has infected its host. The present invention relates to mutants of native Hepatitis C Virus (HCV) RNA polymerase, said mutants imparting improved crystallization properties of the enzyme and enzyme/compound complexes. The present invention also relates to the isolation and identification of an allosteric binding cleft within the HCV NS5B RNA polymerase and its use in the discovery, identification, and characterization of inhibitors of same.

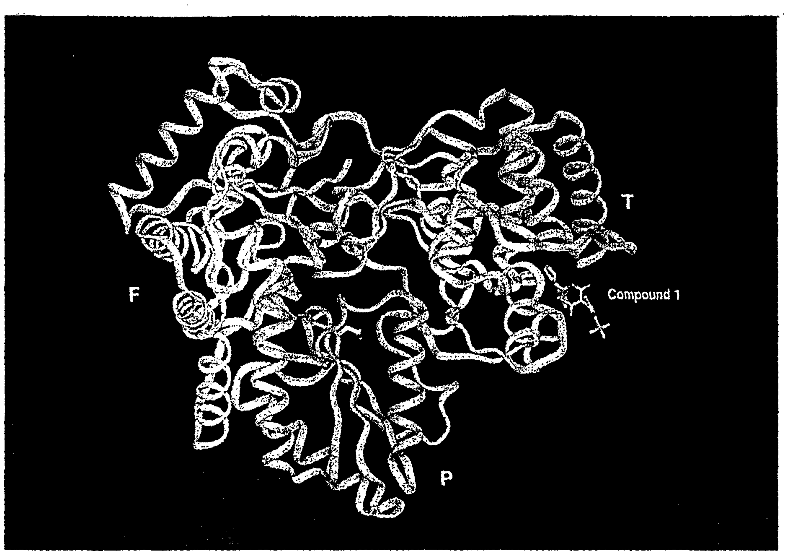


**Fig. 1.**

EP 1 256 628 A2

## Description

### FIELD OF THE INVENTION

The present invention generally relates to viral RNA polymerases, which are essential to viral reproduction and propagation in a host. Viral RNA polymerases are known to play a role in the replication of the virus once it has infected its host. The present invention relates to mutants of native Hepatitis C Virus (HCV) RNA polymerase, said mutants imparting improved crystallization properties of the enzyme and enzyme/compound complexes. The present invention also relates to the isolation and identification of an allosteric binding cleft within the HCV NS5B RNA polymerase and its use in the discovery, identification, and characterization of inhibitors of same.

### BACKGROUND OF THE INVENTION

Non-A, Non-B hepatitis (NANBH) is a transmissible disease that is believed to be virally induced, and is distinguishable from other forms of virus-associated liver disease, such as those caused by hepatitis A (HAV), Hepatitis B (HBV), Hepatitis D (HDV), cytomegalovirus (CMV), or Epstein-Barr virus (EBV). NANBH, labeled Hepatitis C (HCV), has been identified as the primary cause of blood-associated NANBH. Hepatitis C virus transmission occurs primarily via blood-to-blood contact, and appears to be the major form of transfusion-associated hepatitis in a number of countries, including the United States and Japan.

After isolation of the HCV genome and development of an efficient blood-screening system for antibodies against HCV, the incidence of post-transfusion hepatitis by HCV has drastically decreased. However, over 100 million people worldwide are already infected with HCV, and only 20-30% of patients with HCV are sensitive to interferon (IFN) treatment. Furthermore, persistent infection with HCV is also highly associated with the development of liver cirrhosis and hepatocellular carcinoma. The carcinogenesis mechanisms caused by HCV are still poorly understood. The absence of an effective in vitro replication system has hampered the study of HCV and the development of therapeutic measures for HCV infection. However, in vitro translation and gene expression in cells have revealed the properties of several HCV proteins.

HCV is a positive-stranded RNA virus with a linear RNA genome approximately 9.6kb in size (Choo et al. (1989) Science 244:359-362). This genome encodes a single polyprotein of approximately 3010 amino acids and at least ten viral proteins: NH2-C-E1-E2-p7-NS2-NS3-NS4A-NS4B-NS5A-NSSB-COOH (Choo et al. (1991) Proc. Natl. Acad. Sci. USA 88:2451-55; Kaito et al. (1994) J. Gen. Virol. 75:1755-60; Rice (1996) In: Fields, Knipe, Howley (eds) Virology, Raven (New York), pp. 931-960; Takamizawa et al. (1991) J. Virol. 65:1105-13). The individual proteins are released from the polyprotein by both signal peptidases and viral proteases (Grakoui et al. (1993) J. Virol. 67:1385-95; Hijikata et al. (1991) Proc. Natl. Acad. Sci. USA 88:5547-5551; Lin et al. (1994) J. Virol. 68:5063-73). Some or all of the non-structural proteins (NS2, NS3, NS4A, NS4B, NS5A, NS5B) are believed to interact to form the viral replication machinery (Rice, J. Virol. (1996) 70 (6) 3363-71).

The sequence of the NS5B domain protein is highly conserved among the different strains of HCV. NS5B protein is a 65-kD membrane-associated phosphoprotein containing the conserved amino acid motif of RNA-dependent RNA polymerase. NS5B polymerase protein expressed in insect cells has been shown to have a primer-dependent RNA-dependent RNA polymerase activity, which is able to copy full-length HCV RNA transcribed in vitro without addition of other HCV proteins. (Behrens et al. (1996) EMBO J. 15:12-22; Lohmann et al. Science (1998) 71:8416-28. Ishii et al. (1999) Hepatology 29:1227-35). NS5B RNA polymerase is only utilized in the replication of RNA viruses and is considered to play an essential role in viral replication. Therefore, it should be a target for antiviral drugs.

The molecular properties of the NS5B RNA polymerase have not been fully elucidated. The present disclosure describes: 1) mutants of the NS5B RNA polymerase that possess unique properties; 2) mutants that are crystallized; 3) an allosteric binding site contained within the NS5B RNA polymerase and its crystal structure; and, 4) small molecule inhibitors of RNA polymerase. Further, three-dimensional information of the mutated NS5B RNA polymerase and its complex with a chemical compound is described for the first time, enabling elucidation of small molecule inhibitors. Such inhibitors may prove to be valuable new therapeutic entities for the treatment of HCV.

### SUMMARY OF THE INVENTION

The present invention discloses the generation, cloning, and analysis of Hepatitis C virus RNA polymerase located in the NS5B domain of HCV. Single and multiple amino acid mutations were inserted into the NS5B RNA polymerase, which are shown to impart improved and novel crystallization properties.

The present invention further discloses the kinetic characterization and structural determination of the folded NS5B Hepatitis C virus RNA polymerase, including the allosteric binding cleft, said cleft being a small portion of the molecular surface on the RNA polymerase enzyme. This surface region has the appearance of a cleft on the outer

perimeter of the enzyme's "thumb" domain.

It is an object of the invention to provide an isolated, purified polynucleotide which encodes a mutated form of the NS5B RNA polymerase of HCV comprising single or multiple amino acid point mutations, or a fragment thereof, or an active analog thereof.

It is an object of the invention to provide an isolated polypeptide comprising a mutated form of the NS5B RNA polymerase of HCV comprising single or multiple amino acid point mutations, or a fragment thereof, or an active analog thereof.

It is a further object of the invention to provide a crystal structure of the mutated form of the NS5B RNA polymerase of HCV comprising single and multiple amino acid point mutations, or a fragment thereof, or an active analog thereof.

It is another object of the invention to provide an expression vector for producing the mutated form of the NS5B RNA polymerase of HCV comprising single and multiple amino acid point mutations, or a fragment thereof, or an active analog thereof.

It is another object of the invention to provide a bacterial host cell stably or transiently transformed with a polynucleotide encoding a mutated form of the HCV NS5B RNA polymerase comprising single and multiple amino acid point mutations, or a fragment thereof, or an active analog thereof, in a manner allowing expression of the mutated form of the HCV NS5B RNA polymerase.

It is another object of the invention to provide a eukaryotic host cell stably or transiently transformed with a polynucleotide encoding a mutated form of the HCV NS5B RNA polymerase comprising single or multiple amino acid point mutations, or a fragment thereof, or an active analog thereof, in a manner allowing expression of the mutated form of the HCV NS5B RNA polymerase.

It is an object of the invention to provide an isolated, purified polynucleotide which encodes the HCV NS5B RNA polymerase, including the allosteric binding cleft of the or an active analog thereof. The polynucleotide may be natural or recombinant.

It is another object of the invention to provide an isolated polypeptide comprising the allosteric binding cleft of the HCV NS5B RNA polymerase, or a fragment thereof, or an active analog thereof.

It is another object to provide a crystal structure of the HCV NS5B RNA polymerase, including the allosteric binding cleft, or an active analog thereof.

It is another object of the invention to provide an expression vector for producing the HCV NS5B RNA polymerase, including the allosteric binding cleft, in a host cell, or a fragment thereof, or an active analog thereof.

It is another object of the invention to provide a bacterial host cell stably or transiently transformed with a polynucleotide encoding the HCV NS5B RNA polymerase, including the allosteric binding cleft, or a fragment thereof, or an active analog thereof, in a manner allowing expression of the HCV NS5B RNA polymerase, including the allosteric binding cleft.

It is another object of the invention to provide a eukaryotic host cell stably or transiently transformed with a polynucleotide encoding the HCV NS5B RNA polymerase, including the allosteric binding cleft, or a fragment thereof, or an active analog thereof, in a manner allowing expression of the allosteric binding cleft of the HCV NS5B RNA polymerase.

It is another object of the present invention to provide methods for screening candidate compounds based on the molecular structure of the HCV NS5B RNA polymerase, including the allosteric binding cleft derived from x-ray crystallography data to model the binding of said candidate compounds.

These objects as well as others are fully described in detail by the following drawings, detailed description, and claims.

## BRIEF DESCRIPTION OF THE DRAWINGS

Figure 1: Overall view of HCV NS5B polymerase. Domains are denoted F for fingers, P for palm, and T for thumb. The position of bound Inhibitor No. 1 (Compound 1) is also shown on right; the protein structure is essentially the same for inhibitor-bound and apo forms. Active site aspartic acids 318 and 319 are highlighted in center.

Figure 2: Overall view of molecular surface of HCV NS5B polymerase. The domains are colored as thumb (blue and purple), palm (green), and fingers (orange). The enzyme's active site residues are indicated by the red surface at center (Asp 318, Asp 319). Bound Inhibitor No. 1 is shown at left. The purple surface defines the allosteric site, which is a sub-area of the larger thumb domain (blue).

Figure 3: Closer view of surface on HCV NS5B polymerase thumb region (colored blue and purple), showing bound Inhibitor No. 1. The sub-portion of the thumb defined as the allosteric site is colored purple. Since the

site has the appearance of an extended canyon (running horizontal here), it can be assigned features of a "floor" (lighter purple) and "walls" (darker purple). While Inhibitor No. 1 occupies only the central portion of the site, there is clearly room to elaborate this inhibitor for a more complete filling of the canyon.

Figure 4: Stereo view of bound Inhibitor No. 1, identifying residues discussed in the text. The protein is colored yellow, and the inhibitor purple. Distances of hydrogen bonds are indicated.

Figure 5a: Stereo view comparison of alpha-carbon trace for unbound (purple) and inhibitor-bound form (yellow) of HCV NS5B polymerase, with bound Inhibitor No. 1 shown in green. The thumb domain is located within the top one third of the figure. There is little change between apo and inhibitor bound forms of the enzyme, even in the thumb domain.

Figure 5b: Stereo view of the inhibitor binding site of NS5B polymerase, comparing the enzyme before (purple) and after (yellow) the binding of Inhibitor No. 1 (green). Only a few sidechains (Leu 419, Met 423, Leu 497) reorient upon inhibitor binding. There is also a slight shift of residues 495-499 away from the bound inhibitor.

Figure 6: View of the surface on the thumb domain of HCV NS5B polymerase (light blue), showing bound inhibitor Inhibitor No. 1. Positively charged protein side-chains are colored in dark blue (lysine, arginine), negative side-chains in red (aspartic acid, glutamic acid). A nearly continuous patch of positive charge is seen stretching across the thumb domain horizontally just above the inhibitor. This charge involves a number of conserved arginines which are known to be functionally important, and may play a role in interaction with RNA strand(s). Inhibitors bound in the allosteric site canyon, lying alongside this patch, may act by interfering with normal protein-RNA interactions.

Figure 7a: Residues defining the thumb-domain binding site for allosteric inhibitors of HCV NS5B polymerase. The sequence shown is for strain BK. Amino acids in bold have >99% identity among all HCV serotypes, and are also highly conserved between HCV and HGV sequences. Residues in italics are "type-conserved" among HCV and HGV strains, e.g., only a basic side-chain (Lys or Arg) is found at that position, or only a small hydrophobic side-chain (Val, Ile, Leu), etc.

Figure 7b: View similar to Fig.3, but the surface of the NS5B allosteric site (purple) is further colored according to HCV serotype homology: Residues which are invariant among HCV strains are colored yellow, those which are typeconserved among all HCV and HGV strains are colored orange. This shows that a majority of the allosteric site, particularly around bound inhibitor Inhibitor No. 1, is highly conserved among relevant serotypes. Therefore, such an inhibitor should effectively target this allosteric site in most genotypes of HCV and HGV.

Figure 8: The $IC_{50}$ for Inhibitor No. 1 was determined using the assay described in Example 9. The table shows the results of fitting the primary data where m1 = cpm incorporated in the absence of inhibitor, and m2 = $IC_{50.}$

Figure 9: Crystal coordinates of triple mutant (MUT-5) complexed with compound 1.

Figure 10: Crystal coordinates of NS5B polymerase MUT-1.

Figure 11: Crystal coordinates of NS5B polymerase MUT-2

## DETAILED DESCRIPTION OF THE INVENTION

The practice of the present invention generally employs conventional techniques of molecular biology, microbiology, recombinant DNA, and immunology, which are within the skill of the art. Such techniques are explained fully in the literature. See, for example, J. Sambrook et al., "Molecular Cloning: A Laboratory Manual (1989); "DNA Cloning", Vol. I and II (D.N. Glover ed. 1985); "Oligonucleotide Synthesis" (M.J. Gait ed, 1984); "Nucleic Acid Hybridization" (B. D. Hames & S. J. Higgins eds. 1984).

The terms "Hepatitis C Virus" and "HCV" refer to the viral species that is the major etiological agent of Non-A, Non-B Hepatitis (NANBH), the prototype isolate of which was identified by Takamizawa et al., J. Virol., 65:1105-1113 (1991); GenBank reference gi329770, the disclosures of which are incorporated by reference. "HCV" as used herein includes the pathogenic strains capable of causing hepatitis C, and attenuated or mutant strains derived therefrom. There are multiple known strains of HCV. It is known that RNA-containing viruses have relatively high rates of spontaneous mutations. As an RNA virus, it is inherent that there will be multiple strains/isolates, which may be virulent or avirulent, within the HCV species as defined herein.

Due to the evolutionary relationship of the strains or isolates of HCV, putative HCV strains and isolates are identifiable by their homology at the polypeptide level. With respect to the isolates disclosed herein, new HCV strains or isolates are expected to be at least about 40% homologous, some more than about 70% homologous, and some even more than about 90% homologous at the polypeptide level. The techniques for determining amino acid homology are well known in the art. For example, the amino acid sequence may be determined directly and compared to the sequences provided herein. Alternatively the nucleotide sequence of the genomic material of the putative HCV may

be determined (usually via a cDNA intermediate), and then the amino acid sequence encoded therein can be determined, and the corresponding regions compared.

The term "HCV RNA polymerase" or "RNA polymerase" or "NS5B RNA polymerase" refer to an enzyme derived from HCV that enables replication of the virus by synthesizing RNA transcripts off of the RNA viral genome. At least one species of HCV contains a RNA polymerase believed to be substantially encoded by or within SEQ ID No. 1.

The N and C termini of SEQ ID No. 1 are putative, the actual termini being defined by expression and processing in an appropriate host of a DNA construct encoding the entire NS5B domain. It is understood that this sequence may vary from strain to strain, as RNA viruses like HCV are known to exhibit variation. Further, the actual N and C termini may differ from strain to strain as the actual polymerase is cleaved from a precursor polyprotein. Variations in the polymerase amino acid sequence can result in cleavage from the polyprotein at different points. Thus, the amino- and carboxy- termini may differ from strain to strain of HCV.

Structural information of the native three-dimensional configuration of the expressed structural proteins of HCV will lead to a better understanding of the catalytic mechanism behind these proteins. Such information can be obtained once determination of the structure of a protein is generated via X-ray crystallography. Crystal structures of the NS5B polymerase and its complexes with potential inhibitors are important in designing and improving chemical inhibitors to the protein. Variations of the native protein are often necessary to fully enable effective crystallization.

The NS5B RNA polymerase was altered by site-directed mutagenesis, and full RNA polymerase activity was retained while significantly improving the ability to crystallize the protein and obtain the necessary resolution. The amino acid point mutations made in the NS5B RNA polymerase significantly improved the crystal properties, as well as the protein solubility and crystal formation of the protein-compound complexes.

As disclosed herein, point mutations, or single amino acid replacements, were made within the HCV NS5B RNA native polymerase, depicted in SEQ ID No. 1. The point mutations can be made by any known method in the art. Suitable methods include directed mutagenesis by polymerase chain reaction (PCR), a combination of PCR and restriction enzymes specific for methylated DNA (Quickchange by Stratagene, La Jolla, CA), oligonucleotide directed mutagenesis, gene synthesis, and linker scanning mutagenesis. See Current Protocols in Molecular biology, Ausubel et al. John Wiley & Sons, Maniatis.

As disclosed herein, the inventors have also discovered an allosteric binding domain of the NS5B RNA polymerase. This domain serves as a novel and unique binding site for small molecules; such binding in turn serves to inhibit the RNA polymerase enzyme itself. The shape and feature of this binding site or cleft is described, as well as the design of complementary inhibitors that bind tightly to it. Such inhibitors can be developed into drug candidates for treating HCV.

Methods of assaying for a candidate compound and its ability to interact with the RNA polymerase allosteric binding cleft are also described. Specifically, expression of the allosteric binding cleft is accomplished as taught herein. The binding cleft is exposed to a candidate inhibitor compound, and evaluation of the interaction is accomplished by standard means. A preferred method for evaluating the HCV RNA NS5B polymerase allosteric binding site is to measure the enzyme's ability to perform primer/template-directed transcription in the presence and absence of candidate inhibitor compounds.

The invention also teaches methods of drug design. Using the methods taught by the examples herein, one may design drugs that will fit into the allosteric binding cleft and thereby inhibit the HCV NS5B RNA polymerase. The crystal structure of the inhibitor molecule enables one of ordinary skill to apply an iterative process whereby various molecule structures are applied to a computer generated model to identity potential agonists or antagonists. A preferred method includes: (1) crystallizing the HCV NS5B RNA polymerase including the allosteric binding cleft; (2) applying the data generated from the crystal structure to a computer algorithm which generates a model of said HCV NS5B RNA polymerase including the allosteric binding cleft; and, (3) determining molecular structures that will fit and bind to the allosteric binding cleft.

The novel binding cleft is found on the outer perimeter of the "thumb" domain of the RNA polymerase, far from the central cavity containing the enzyme's active site, or the RNA synthesis location. See Figures 1 through 6. The fact that small molecules bound within the cleft can still inhibit the enzyme's function makes this cleft an "allosteric" site, i.e. inhibitors operate indirectly to interfere with the enzyme's function.

The amino acids that form this allosteric site, and the shape/features of this site, are unique to the HCV RNA NS5B polymerase enzyme, and are conserved among most Flaviviridae. The only detailed structural motifs common among all polymerases are found within the central cavity where RNA strand synthesis is performed. Therefore, inhibitors designed against the HCV allosteric site will be highly specific toward viral polymerase but not cellular polymerases, greatly decreasing the likelihood of drug toxicity.

Peptides, Proteins, and Antibodies

The present invention provides isolated peptide and protein molecules that consist of, consist essentially of,

or are comprised of the amino acid sequences of the RNA polymerase peptides encoded by the nucleic acid sequences disclosed in SEQ ID Nos. 2, 6, 7 or 8, as well as all obvious variants of these peptides that are within the art to make and use. These variants are defined and described in detail below.

As used herein, the terms 'polymerase,' 'RNA polymerase,' and 'NS5B RNA polymerase' refer to enzymes that enable replication of the HCV in a host.

As used herein, a peptide is said to be "isolated" or "purified" when it is substantially free of cellular or viral material or free of chemical precursors or other chemicals. The peptides of the present invention can be purified to homogeneity or other high degrees of purity. The level of purification will be based on the intended use. The critical feature is that the preparation allows for the desired function of the peptide, even if in the presence of considerable amounts of other components.

In some uses, "substantially free of cellular or viral material" includes preparations of the peptide having less than about 30% (by dry weight) of other proteins (i.e. contaminating protein), less than about 20% other proteins, less than about 10% other proteins, or less than about 5% other proteins. Additionally, when the peptide is produced using recombinant techniques, it can be substantially free of culture medium, i.e. culture medium represents less than about 20% of the volume of the protein preparation.

The isolated RNA polymerase described herein can be purified from viruses that naturally express it, purified from cells that have been altered to express it (recombination), or synthesized using known protein synthesis methods. For example, a nucleic acid molecule encoding the protein RNA polymerase is cloned into an expression vector, the expression vector introduced into a host cell and the protein expressed in the host cell. The protein can then be isolated from the cells by an appropriate purification scheme using standard protein purification techniques. Many of these techniques are described in detail below.

The present invention also provides catalytically active variants of the peptides of the present invention, such as allelic/sequence variants of the peptides, non-naturally occurring recombinantly derived variants of the peptides, and orthologs and paralogs of the peptides. Such variants can be generated using techniques that are known by those skilled in the fields of recombinant nucleic acid technology and protein biochemistry.

Such variants can readily be identified/made using molecular techniques and the sequence information disclosed herein. Further, such variants can readily be distinguished from other peptides based on sequence and/or structural homology to the peptides of the present invention. The degree of homology/identity present will be based primarily on whether the peptide is a functional (active) variant or non-functional (inactive) variant, the amount of divergence present in the paralog family and the evolutionary distance between the orthologs.

To determine the percent identity of two amino acid sequences or two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 15% or more of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid 'identity' is equivalent to amino acid or nucleic acid 'homology'). The percent identity between the two sequences is a function of the number of identical positions shared by the sequences, taking into account the number of gaps, and the length of each gap, which need to be introduced for optimal alignment of the two sequences. The comparison of sequences and determination of percent identity and similarity between two sequences can be accomplished using a mathematical algorithm. (Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part 1, Griffin, A. M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). In a preferred embodiment, the percent identity between two amino acid sequences is determined using the Needleman and Wunsch (J. Mol. Biol. (48):444-453 (1970)) algorithm which has been incorporated into commercially available computer programs, such as GAP in the GCG software package, using either a Blossom 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences can be determined using the commercially available computer programs including the PILEUP, PRETTY, or GAP program in the GCG software package (Devereux, J., et al., Nucleic Acids Res. 12(1):387 (1984)), the NWS gap DNA CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Meyers and W. Miller (CABIOS, 4: 11-17 (1989)) which has been incorporated into commercially available computer programs, such as ALIGN (version 2.0), using a PAM120 weight residue table, a gap length penalty of 12 and a gap penalty of 4.

The nucleic acid and protein sequences of the present invention can further be used as a "query sequence"

to perform a search against sequence databases to, for example, identify other family members or related sequences. Such searches can be performed using commercially available search engines, such as the PSI-BLAST, NBLAST and XBLAST programs (version 2.0) of Altschul, et al. (J. Mol. Biol. 215:403-10 (1990)). Nucleotide searches can be performed with such programs to obtain nucleotide sequences homologous to the nucleic acid molecules of the invention. Protein searches can be performed with such programs to obtain amino acid sequences homologous to the proteins of the invention. To obtain gapped alignments for comparison purposes, Gapped BLAST can be utilized as described in Altschul et al. (Nucleic Acids Res. 25(17):3389-3402 (1997)).

Full-length clones comprising one of the peptides of the present invention can readily be identified as having complete sequence identity to one of the RNA polymerases of the present invention..

Variants of a peptide can readily be identified as having some degree of sequence homology/identity to at least a portion of the RNA polymerase peptide provided herein. As used herein, two proteins (or a region of the proteins) have significant homology when the amino acid sequences are typically at least about 60% homologous.

Orthologs of a RNA polymerase peptide can readily be identified as having some degree of significant sequence homology/identity to at least a portion of the RNA polymerase peptide as well as being encoded by a gene from another organism. Preferred orthologs will be isolated from other viral species, particularly other Hepatitis species, including but not limited to Hepatitis A virus, Hepatitis B virus, Hepatitis D virus, Hepatitis G virus, and other nonA, nonB Hepatitis species, such as Rhinovirus, Yellow fever, Dengue fever, or Western Nile. Hepatitis virus is in the family of flavivirus, therefore, other members of this family will have RNA polymerases that will have significant homology to the HCV RNA polymerase. Such polymerases are functionally and structurally related, although their sequence identity might be lower than the identities among eukaryotic species. Furthermore, although overall homology might be low among sequences, the binding cleft homology will be relatively high. Non-naturally occurring variants of the RNA polymerase of the present invention can readily be generated using recombinant techniques. Such variants include, but are not limited to deletions, additions and substitutions in the amino acid sequence of the RNA polymerase. For example, one class of substitutions is conserved amino acid substitutions. Such substitutions are those that substitute a given amino acid in a RNA polymerase peptide by another amino acid of like characteristics. Typically seen as conservative substitutions are the replacements: 1) one for another among the aliphatic amino acids Ala, Val, Leu, and Ile; 2) interchange of the hydroxyl residues Ser and Thr; 3) exchange of the acidic residues Asp and Glu; 4) substitution between the amide residues Asn and Gln; exchange of the basic residues Lys and Arg; and, 5) replacements among the aromatic residues Phe, Tyr. Guidance concerning which amino acid changes are likely to be phenotypically silent are found in Bowie et al., Science 247:1306-1310 (1990).

Variant RNA polymerases can be fully functional or can lack function in one or more activities. Fully functional variants typically contain only conservative variation or variation in non-critical residues or in non-critical regions. Functional variants can also contain substitution of similar amino acids, which result in no change or an insignificant change in function. Alternatively, such substitutions may positively or negatively affect function to some degree.

Non-functional variants typically contain one or more non-conservative amino acid substitutions, deletions, insertions, inversions, or truncation or a substitution, insertion, inversion, or deletion in a critical residue or critical region.

Amino acids that are essential for function can be identified by methods known in the art, such as site-directed mutagenesis or alanine-scanning mutagenesis (Cunningham et al., Science 244:1081-1085 (1989)). The latter procedure introduces single alanine mutations at every residue in the molecule. The resulting mutant molecules are then tested for biological activity such as receptor binding or in vitro proliferative activity. Sites that are critical for binding can also be determined by structural analysis such as x-ray crystallography, nuclear magnetic resonance or photoaffinity labeling (Smith et al., J. Mol. Biol. 224:899-904 (1992); de Vos et al. Science 255:306-312 (1992)). Accordingly, the protein RNA polymerases of the present invention also encompass derivatives or analogs in which a substituted amino acid residue is not one encoded by the genetic code; in which a substituent group is included; in which the mature polypeptide is fused with another compound, such as a compound to increase the half-life of the polypeptide (for example, polyethylene glycol); or in which the additional amino acids are fused to the mature polypeptide, such as a leader or secretory sequence or a sequence for purification of the mature polypeptide or a pro-protein sequence.

The present invention further provides for functional, active fragments of the HCV RNA polymerase. As used herein, a fragment comprises at least 8 or more contiguous amino acid residues from the protein RNA polymerase. Such fragments can be chosen based on the ability to retain one or more of the biological activities of the RNA polymerase or could be chosen for the ability to perform a function, e.g. act as an immunogen. Particularly important fragments are catalytically active fragments, i.e. peptides that are, for example, about 8 or more amino acids in length. Such fragments will typically comprise a domain or motif of the RNA polymerase, e.g., active site or binding site. Further fragments contemplated by the present invention include, but are not limited to, domain or motif containing fragments, soluble peptide fragments, and fragments containing immunogenic structures. Predicted domains and functional sites available to those of skill in the art (e.g., by PROSITE analysis).

Polypeptides often contain amino acids other than the 20 amino acids commonly referred to as the 20 naturally occurring amino acids. Further, many amino acids (including the terminal amino acids) may be modified by natural

processes, such as processing and other post-translational modifications, or by chemical modification techniques known in the art. Common modifications that occur naturally in polypeptides are described in basic texts, detailed monographs, and research literature. Moreover, such modifications are known to those skilled in the art.

Known modifications include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, phenylation, racemization, selenoylation, sulfation, transfer-RNA mediated addition of amino acids to proteins such as arginylation, and ubiquitination. Such modifications are not only known to skilled artisans, but are described in great detail in scientific literature. Several particularly common modifications, glycosylation, lipid attachment, sulfation, gamma-carboxylation of glutamic acid residues, hydroxylation and ADP-ribosylation, for instance, are described in the most basic texts, such as Proteins - Structure and Molecular Properties, 2nd Ed., T.E. Creighton, W. H. Freeman and Company, New York (1993). Additionally, many detailed reviews are available on this subject, for example Wold, F., Posttranslational Covalent Modification of Proteins, B.C. Johnson, Ed., Academic Press, New York 1-12 (1983); Seifter et al. (Meth. Enzymol. 182: 626-646 (1990)) and Rattan et al. (Ann. N.Y. Acad. Sci. 663:48-62 (1992)).

The peptides of the present invention can be attached to heterologous sequences to form chimeric or fusion proteins. Such chimeric and fusion proteins comprise a peptide operatively linked to a heterologous protein having an amino acid sequence not substantially homologous to the RNA polymerase peptide. "Operatively linked" indicates that the peptide and the heterologous protein are fused in-frame. The heterologous protein can be fused to the N-terminus or C-terminus of the RNA polymerase peptide. The two peptides linked in a fusion peptide are typically derived from two independent sources, and therefore a fusion peptide comprises two linked peptides not normally found linked in nature. The two peptides may be from the same or different genome.

In some uses, the fusion protein does not affect the activity of the peptide *per se.* For example, the fusion protein can include, but is not limited to, enzymatic fusion proteins, for example beta-galactosidase fusions, yeast two-hybrid GAL fusions, poly-His fusions, MYC-tagged, HI-tagged and Ig fusions. Such fusion proteins, particularly poly-His fusions, can facilitate the purification of recombinant RNA polymerase peptide. Moreover, expression and/or secretion of a protein can be increased by using a heterologous signal sequence in certain host cells, for example in mammalian host cells.

A chimeric or fusion protein can be produced by standard recombinant DNA techniques. For example, DNA fragments coding for the different protein sequences are ligated together in-frame in accordance with conventional techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence (see Ausubel et al., Current Protocols in Molecular Biology, 1992). Moreover, many expression vectors are commercially available that already encode a fusion moiety (e.g., a GST protein). A RNA polymerase peptide-encoding nucleic acid can be cloned into such an expression vector such that the fusion moiety is linked in-frame to the RNA polymerase peptide.

Herein, the term 'antibody' refers to a polypeptide or group of polypeptides which are comprised of at least one antibody combining site or binding domain, said binding domain or combining site formed from the folding of variable domains of an antibody molecule to form three dimensional binding spaces with an internal surface shape and charge distribution complementary to the features of an antigen epitope. The term encompasses immunoglobulin molecules and immunologically active portions of immunoglobulin molecules, such as molecules that contain an antibody combining site or paratope. Exemplary antibody molecules are intact immunoglobulin molecules, substantially intact immunoglobulin molecules and portions of an immunoglobulin molecule, including those known in the art as Fab, FabB, $F(abB)_2$ and F(v).

Nucleic Acids and Polynucleotides

The present invention provides isolated nucleic acid molecules that encode the functional, active RNA polymerases of the present invention. Such nucleic acid molecules will consist of, consist essentially of, or comprise a nucleotide sequence that encodes one of the RNA polymerase peptides of the present invention, or HCV strain variants thereof.

As used herein, an "isolated" nucleic acid molecule is one that is separated from other nucleic acid present in the natural source of the nucleic acid. Preferably, an "isolated" nucleic acid is free of sequences which naturally flank the nucleic acid (i.e., sequences located at the 5' and 3' ends of the nucleic acid) in the genomic DNA or cDNA of the organism from which the nucleic acid is derived. However, there can be some flanking nucleotide sequences, for

example up to about 5KB, particularly contiguous peptide encoding sequences and peptide encoding sequences within the same gene but separated by introns in the genomic sequence. The important point is that the nucleic acid is isolated from remote and unimportant flanking sequences such that it can be subjected to the specific manipulations described herein such as recombinant expression, preparation of probes and primers, and other uses specific to the nucleic acid sequences.

Moreover, an "isolated" nucleic acid molecule, such as a cDNA molecule, can be substantially free of other cellular material, or culture medium when produced by recombinant techniques, or chemical precursors or other chemicals when chemically synthesized. However, the nucleic acid molecule can be fused to other coding or regulatory sequences and still be considered isolated.

For example, recombinant DNA molecules contained in a vector are considered isolated. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the isolated DNA molecules of the present invention. Isolated nucleic acid molecules according to the present invention further include such molecules produced synthetically.

The preferred classes of nucleic acid molecules that are comprised of the nucleotide sequences of the present invention are the full-length cDNA molecules and genes and genomic clones since some of the nucleic acid molecules provided in SEQ ID Nos. 2, 5, and 6 are fragments of the complete gene that exists in nature. A brief description of how various types of these nucleic acid molecules can be readily made/isolated is provided herein.

Full-length genes may be cloned from known sequence using any one of a number of methods known in the art. For example, a method that employs XL-PCR (Perkin-Elmer, Foster City, Calif.) to amplify long pieces of DNA may be used. Other methods for obtaining full-length sequences are known in the art.

The isolated nucleic acid molecules can encode the functional, active RNA polymerase plus additional amino or carboxyl-terminal amino acids, such as those that facilitate protein trafficking, prolong or shorten protein half-life or facilitate manipulation of a protein for assay or production, among other things. The isolated nucleic acid molecules include, but are not limited to, the sequence encoding the active RNA polymerase alone or in combination with coding sequences, such as a leader or secretory sequence (e.g., a pre-pro or pro-protein sequence), the sequence encoding the active RNA polymerase, with or without the additional coding sequences, plus additional non-coding sequences, for example introns and non-coding 5' and 3' sequences such as transcribed but non-translated sequences that play a role in transcription, mRNA processing (including splicing and polyadenylation signals), ribosome binding and stability of mRNA. In addition, the nucleic acid molecule may be fused to a marker sequence encoding, for example, a peptide that facilitates purification.

Isolated nucleic acid molecules can be in the form of RNA, such as mRNA, or in the form of DNA, including cDNA and genomic DNA, obtained by cloning or produced by chemical synthetic techniques or by a combination thereof. The nucleic acid, especially DNA, can be double-stranded or single-stranded. Single-stranded nucleic acid can be the coding strand (sense strand) or the non-coding strand (anti-sense strand).

The invention further provides nucleic acid molecules that encode functional fragments or variants of the active RNA polymerases of the present invention. Such nucleic acid molecules may be naturally occurring, such as allelic variants (same locus) and orthologs (different organism), or may be constructed by recombinant DNA methods or by chemical synthesis. Such non-naturally occurring variants may be made by mutagenesis techniques, including those applied to nucleic acid molecules, cells, or organisms. Accordingly, as discussed above, the variants can contain nucleotide substitutions, deletions, inversions and insertions. Variation can occur in either or both the coding and non-coding regions. The variations can produce both conservative and non-conservative amino acid substitutions.

A fragment comprises a contiguous nucleotide sequence greater than 12 or more nucleotides. Further, a fragment could be at least 30, 40, 50, 100, 250 or 500 nucleotides in length. The length of the fragment will be based on its intended use. For example, the fragment can encode epitope bearing regions of the peptide, or can be useful as DNA probes and primers. Such fragments can be isolated using the known nucleotide sequence to synthesize an oligonucleotide probe. A labeled probe can then be used to screen a cDNA library, genomic DNA library, or mRNA to isolate nucleic acid corresponding to the coding region. Further, primers can be used in PCR reactions to clone specific regions of gene.

A probe/primer typically comprises substantially a purified oligonucleotide or oligonucleotide pair. The oligonucleotide typically comprises a region of nucleotide sequence that hybridizes under stringent conditions to at least about 12, 20, 25, 40, 50 or more consecutive nucleotides.

As used herein, the term "hybridizes under stringent conditions" is intended to describe conditions for hybridization and washing under which nucleotide sequences encoding a peptide at least 50-55% homologous to each other typically remain hybridized to each other. The conditions can be such that sequences at least about 65%, at least about 70%, or at least about 75% or more homologous to each other typically remains hybridized to each other. Such stringent conditions are known to those skilled in the art and can be found in Current Protocols in Molecular Biology, John Wiley & Sons, N.Y. (1989), 6.3.1-6.3.6. One example of stringent hybridization conditions are hybridization in 6X sodium

chloride/sodium citrate (SSC) at about 45°C, followed by one or more washes in 0.2 X SSC, 0.1% SDS at 50-65C.

The nucleic acid molecules of the present invention are useful for probes, primers, chemical intermediates, and in biological assays. The nucleic acid molecules are useful as a hybridization probe for cDNA and genomic DNA to isolate full-length cDNA and genomic clones encoding the peptide described herein and to isolate cDNA and genomic clones that correspond to variants (alleles, orthologs, etc.) producing the same or related peptides described herein.

The nucleic acid molecules are also useful as primers for PCR to amplify any given region of a nucleic acid molecule and are useful to synthesize antisense molecules of desired length and sequence.

The nucleic acid molecules are also useful for constructing recombinant vectors. Such vectors include expression vectors that express a portion of, or all of, the peptide sequences. Vectors also include insertion vectors, used to integrate into another nucleic acid molecule sequence, such as into the cellular genome, to alter in situ expression of a gene and/or gene product. For example, an endogenous coding sequence can be replaced via homologous recombination with all or part of the coding region containing one or more specifically introduced mutations.

The nucleic acid molecules are also useful for expressing antigenic portions of the proteins.

The nucleic acid molecules are also useful as probes for determining the chromosomal positions of the nucleic acid molecules by means of in situ hybridization methods.

The nucleic acid molecules are also useful for designing ribozymes corresponding to all, or a part, of the mRNA produced from the nucleic acid molecules described herein.

The nucleic acid molecules are also useful for constructing host cells expressing a part, or all, of the nucleic acid molecules and peptides.

The nucleic acid molecules are also useful for constructing transgenic animals expressing all, or a part, of the nucleic acid molecules and peptides.

The nucleic acid molecules are also useful for making vectors that express part, or all, of the peptides.

The nucleic acid molecules are also useful as hybridization probes for determining the presence, level, form and distribution of nucleic acid expression. Accordingly, the probes can be used to detect the presence of, or to determine levels of, a specific nucleic acid molecule in cells, tissues, and in organisms. The nucleic acid whose level is determined can be DNA or RNA. Accordingly, probes corresponding to the peptides described herein can be used to assess expression and/or gene copy number in a given cell, tissue, or organism. These uses are relevant for diagnosis of disorders involving an increase or decrease in RNA polymerase protein expression relative to normal results.

In vitro techniques for detection of mRNA include Northern hybridizations and in situ hybridizations. In vitro techniques for detecting DNA include Southern hybridizations and in situ hybridization.

Probes can be used as a part of a diagnostic test kit for identifying cells or tissues that express a RNA polymerase protein, such as by measuring a level of a receptor-encoding nucleic acid in a sample of cells from a subject e.g., mRNA or genomic DNA, or determining if a receptor gene has been mutated.

Vectors and Host Cells

The invention also provides vectors containing the nucleic acid molecules described herein. The term "vector" refers to a vehicle, preferably a nucleic acid molecule, that can transport the nucleic acid molecules. When the vector is a nucleic acid molecule, the nucleic acid molecules are covalently linked to the vector nucleic acid. With this aspect of the invention, the vector includes a plasmid, single or double stranded phage, a single or double stranded RNA or DNA viral vector, or artificial chromosome, such as a BAC, PAC, YAC, OR MAC. Various expression vectors can be used to express polynucleotide encoding the active HCV RNA polymerase.

A vector can be maintained in the host cell as an extrachromosomal element where it replicates and produces additional copies of the nucleic acid molecules. Alternatively, the vector may integrate into the host cell genome and produce additional copies of the nucleic acid molecules when the host cell replicates.

The invention provides vectors for the maintenance (cloning vectors) or vectors for expression (expression vectors) of the nucleic acid molecules. The vectors can function in prokaryotic or eukaryotic cells or in both (shuttle vectors).

Expression vectors contain cis-acting regulatory regions that are operably linked in the vector to the nucleic acid molecules such that transcription of the nucleic acid molecules is allowed in a host cell. The nucleic acid molecules can be introduced into the host cell with a separate nucleic acid molecule capable of affecting transcription. Thus, the second nucleic acid molecule may provide a trans-acting factor interacting with the cis-regulatory control region to allow transcription of the nucleic acid molecules from the vector. Alternatively, a trans-acting factor may be supplied by the host cell. Finally, a trans-acting factor can be produced from the vector itself. It is understood, however, that in some embodiments, transcription and/or translation of the nucleic acid molecules can occur in a cell-free system.

The nucleic acid molecules described herein can be operably linked to regulatory sequences that direct mRNA transcription. These include, but are not limited to, the left promoter from bacteriophage $\lambda$, the lac, TRP, and TAC promoters from *E. coli,* the early and late promoters from SV40, the CMV immediate early promoter, the adenovirus

early and late promoters, and retrovirus long-terminal repeats.

In addition to control regions that promote transcription, expression vectors may also include regions that modulate transcription, such as repressor binding sites and enhancers. Examples include the SV40 enhancer, the cytomegalovirus immediate early enhancer, polyoma enhancer, adenovirus enhancers, and retrovirus LTR enhancers.

In addition to containing sites for transcription initiation and control, expression vectors can also contain sequences necessary for transcription termination, and a ribosome binding site for translation in the transcribed region. Other regulatory control elements for expression include initiation and termination codons as well as polyadenylation signals. The person of ordinary skill in the art would be aware of the numerous regulatory sequences that are useful in expression vectors. Such regulatory sequences are described, for example, in Sambrook *et al.,* (*Molecular Cloning: A Laboratory Manual. 2nd. ed.,* Cold Spring Harbor Laboratory Press, Cold Spring Harbor, NY, (1989)).

A variety of expression vectors can be used to express a nucleic acid molecule. Such vectors include chromosomal, episomal, and virus-derived vectors, for example vectors derived from bacterial plasmids, from bacteriophage, from yeast episomes, from yeast chromosomal elements, including yeast artificial chromosomes, from viruses such as baculoviruses, papovaviruses such as SV40, Vaccinia viruses, adenoviruses, poxviruses, pseudorabies viruses, and retroviruses. Vectors may also be derived from combinations of these sources such as those derived from plasmid and bacteriophage genetic elements, eg. cosmids and phagemids. Appropriate cloning and expression vectors for prokaryotic and eukaryotic hosts are described in Sambrook *et al., Molecular Cloning: A Laboratory Manual. 2nd. ed.,* Cold Spring Harbor Laboratory Press, Cold Spring Harbor, NY, (1989).

The regulatory sequence may provide constitutive expression in one or more host cells (i.e. tissue specific) or may provide for inducible expression in one or more cell types such as by temperature, nutrient additive, or exogenous factor such as a hormone or other ligand. A variety of vectors providing constitutive and inducible expression in prokaryotic and eukaryotic hosts are known in the art.

The nucleic acid molecules can be inserted into the vector nucleic acid by well-known methodology. Generally, the DNA sequence that will ultimately be expressed is joined to an expression vector by cleaving the DNA sequence and the expression vector with one or more restriction enzymes and then ligating the fragments together. Procedures for restriction enzyme digestion and ligation are known in the art.

The vector containing the appropriate nucleic acid molecule can be introduced into an appropriate host cell for propagation or expression using well-known techniques. Bacterial cells include, but are not limited to, *E. coli*, *Streptomyces*, and *Salmonella typhimurium*. Eukaryotic cells include, but are not limited to, yeast, insect cells such as *Drosophila*, animal cells such as COS and CHO cells, and plant cells.

As described herein, it may be desirable to express a peptide of the present invention as a fusion protein. Accordingly, the invention provides fusion vectors that allow for the production of such peptides. Fusion vectors can increase the expression of a recombinant protein, increase the solubility of the recombinant protein, and aid in the purification of the protein by acting for example as a ligand for affinity purification. A proteolytic cleavage site may be introduced at the junction of the fusion moiety so that the desired peptide can ultimately be separated from the fusion moiety. Proteolytic enzymes include, but are not limited to, factor Xa, thrombin, and enteroRNA polymerase. Typical fusion expression vectors include pGEX (Smith *et al., Gene 67*:31-40 (1988)), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein. Examples of suitable inducible non-fusion *E. coli* expression vectors include pTrc (Amann *et al., Gene 69*:301-315 (1988)) and pET 11d (Studier *et al., Gene Expression Technology: Methods in Enzymology 185*:60-89 (1990)).

Recombinant protein expression can be maximized in a bacterial host cell by providing a genetic background, wherein the host cell has an impaired capacity to proteolytically cleave the recombinant protein. (Gottesman, S., *Gene Expression Technology: Methods in Enzymology* 185, Academic Press, San Diego, California (1990) 119-128). Alternatively, the sequence of the nucleic acid molecule of interest can be altered to provide preferential codon usage for a specific host cell, for example *E. coli.* (Wada *et al.*, *Nucleic Acids Res. 20*:2111-2118 (1992)).

The nucleic acid molecules can also be expressed by expression vectors that are operative in yeast. Examples of vectors for expression in yeast e.g., *S. cerevisiae* include pYepSec1 (Baldari, *et al., EMBO J. 6*:229-234 (1987)), pMFa (Kurjan *et al., Cell 30*:933-943(1982)), pJRY88 (Schultz *et al., Gene 54*:113-123 (1987)), and pYES2 (Invitrogen Corporation, San Diego, CA).

The nucleic acid molecules can also be expressed in insect cells using, for example, baculovirus expression vectors. Baculovirus vectors available for expression of proteins in cultured insect cells (e.g., Sf 9 cells) include the pAc series (Smith *et al., Mol. Cell Biol. 3*:2156-2165 (1983)) and the pVL series (Lucklow *et al., Virology 170*:31-39 (1989)).

In certain embodiments of the invention, the nucleic acid molecules described herein are expressed in mammalian cells using mammalian expression vectors. Examples of mammalian expression vectors include pCDM8 (Seed, B. *Nature 329*:840(1987)) and pMT2PC (Kaufman *et al., EMBO J. 6*:187-195 (1987)).

The expression vectors listed herein are provided by way of example only of the well-known vectors available

to those of ordinary skill in the art that would be useful to express the nucleic acid molecules. Preferred vectors include the pET28a (Novagen, Madison, WI), pAcSG2 (Pharmingen, San Diego, CA), and pFastBac (Life Technologies, Gaithersburg, MD). The person of ordinary skill in the art would be aware of other vectors suitable for maintenance, propagation or expression of the nucleic acid molecules described herein. These are found for example in Sambrook, J., Fritsh, E. F., and Maniatis, T. *Molecular Cloning: A Laboratory Manual. 2nd, ed., Cold Spring Harbor Laboratory*, Cold Spring Harbor Laboratory Press, Cold Spring Harbor, NY, 1989.

The invention also encompasses vectors in which the nucleic acid sequences described herein are cloned into the vector in reverse orientation, but operably linked to a regulatory sequence that permits transcription of antisense RNA. Thus, an antisense transcript can be produced to all, or to a portion, of the nucleic acid molecule sequences described herein, including both coding and non-coding regions. Expression of this antisense RNA is subject to each of the parameters described above in relation to expression of the sense RNA (regulatory sequences, constitutive or inducible expression, tissue-specific expression).

The invention also relates to recombinant host cells containing the vectors described herein. Host cells therefore include prokaryotic cells, lower eukaryotic cells such as yeast, other eukaryotic cells such as insect cells, and higher eukaryotic cells such as mammalian cells. Preferred host cells of the instant invention include *E. coli* and Sf9.

The recombinant host cells are prepared by introducing the vector constructs described herein into the cells by techniques readily available to the person of ordinary skill in the art. These include, but are not limited to, calcium phosphate transfection, DEAE-dextran-mediated transfection, cationic lipid-mediated transfection, electroporation, transduction, infection, lipofection, and other techniques such as those found in Sambrook, *et al.* (*Molecular Cloning: A Laboratory Manual. 2nd, ed., Cold Spring Harbor Laboratory*, Cold Spring Harbor Laboratory Press, Cold Spring Harbor, NY, 1989).

Host cells can contain more than one vector. Thus, different nucleotide sequences can be introduced on different vectors of the same cell. Similarly, the nucleic acid molecules can be introduced either alone or with other nucleic acid molecules that are not related to the nucleic acid molecules such as those providing trans-acting factors for expression vectors. When more than one vector is introduced into a cell, the vectors can be introduced independently, co-introduced or joined to the nucleic acid molecule vector.

In the case of bacteriophage and viral vectors, these can be introduced into cells as packaged or encapsulated virus by standard procedures for infection and transduction. Viral vectors can be replication-competent or replication-defective. In the case in which viral replication is defective, replication will occur in host cells providing functions that complement the defects.

Vectors generally include selectable markers that enable the selection of the subpopulation of cells that contain the recombinant vector constructs. The marker can be contained in the same vector that contains the nucleic acid molecules described herein or may be on a separate vector. Markers include tetracycline or ampicillin-resistance genes for prokaryotic host cells and dihydrofolate reductase or neomycin resistance for eukaryotic host cells. However, any marker that provides selection for a phenotypic trait will be effective.

While the active protein RNA polymerases can be produced in bacteria, yeast, mammalian cells, and other cells under the control of the appropriate regulatory sequences, cell- free transcription and translation systems can also be used to produce these proteins using RNA derived from the DNA constructs described herein.

Where secretion of the peptide is desired, appropriate secretion signals are incorporated into the vector. The signal sequence can be endogenous to the peptides or heterologous to these peptides.

It is also understood that depending upon the host cell in recombinant production of the peptides described herein, the peptides can have various glycosylation patterns, depending upon the cell, or maybe non-glycosylated as when produced in bacteria. In addition, the peptides may include an initial modified methionine in some cases as a result of a host-mediated process.

The recombinant host cells expressing the peptides described herein have a variety of uses. First, the cells are useful for producing a RNA polymerase protein or peptide that can be further purified to produce desired amounts of RNA polymerase protein or fragments. Thus, host cells containing expression vectors are useful for peptide production.

Host cells are also useful for conducting cell-based assays involving the RNA polymerase protein or RNA polymerase protein fragments. Thus, a recombinant host cell expressing a native RNA polymerase protein is useful for assaying compounds that stimulate or inhibit RNA polymerase protein function.

Host cells are also useful for identifying RNA polymerase protein mutants in which these functions are affected. If the mutants naturally occur and give rise to a pathology, host cells containing the mutations are useful to assay compounds that have a desired effect on the mutant RNA polymerase protein (for example, stimulating or inhibiting function) which may not be indicated by their effect on the native RNA polymerase protein.

The Allosteric Binding Site

The "allosteric binding site" on the NS5B polymerase has the general appearance of a long cleft or canyon spanning across most of the width of the thumb domain, and is located on the outer perimeter of the protein. Fig. 2 shows the NS5B triple-mutant structure with Inhibitor No. 1 bound, which specifically highlights (the surface of) residues of the thumb which we define as the allosteric site (in purple). Fig.3 is a closer, more direct view into the allosteric site. This canyon-like site has general features of a "floor" (colored light purple) and "walls" (colored dark purple). The floor and walls are defined here in such a way that they extend beyond the physical boundaries of Inhibitor No. 1. This extended definition allows for chemical elaboration of Inhibitor No. 1, which will provide additional protein/inhibitor contacts. The approximate dimensions of the canyon are 30 Å long by 10 Å wide and 10 Å deep.

We define thumb-domain amino acids forming the surface of the allosteric site as follows: Residues 374-378, 416-427, 469-490, 493-501, and 524-536. More specifically, the "floor" of the canyon-like site is formed primarily by residues 416-427 (involving helix αT3), residues 469-474, residues 485 and 489 (from helix αT5), and residues 493-494. The "walls" of the canyon-like site are formed primarily from residues 374-378 (involving beta strand βT1), residues 475-484, 486-488, 490 (involving helix αT5), residues 495-501 (involving helix αT6), and loop 524-536.

Further, the inventors have elucidated interactions between Inhibitor No. 1 and the HCV NS5B polymerase allosteric binding site. Bound Inhibitor No. 1 occupies the central portion of an extended, canyon-like allosteric binding site (defined above). A detailed view of interactions is shown in Fig. 4. The most intimate interaction involves the compound's cyclopentyl ring, which fits into a small, complementary, and hydrophobic pocket defined by mainchain and sidechain atoms of Met 423, Trp 528, Leu 419, Tyr 477, and Arg 422 (alkyl part of sidechain). One edge of the hydroxyphenyl ring of Inhibitor No. 1 rests on sidechains Met 423, Leu 419, Leu 489, and Val 485 (the "floor" of the canyon); the ring's faces are surrounded by sidechains Leu 497 and Ile 482 (the "walls" of the canyon). The central pyranone ring of Inhibitor No. 1 makes the compound's only polar interactions with the protein: Its hydroxy group makes a hydrogen bond with the backbone amide of Ser 476 directly (distance 2.8 Å), and with the amide of Tyr 477 indirectly through a water molecule. The carbonyl of Inhibitor No. 1's central ring makes a water-mediated hydrogen bond with Arg 501. The phenylsulfanyl ring of Inhibitor No. 1 has the fewest interactions, e.g., parallel stacking with the imidazole of His 475.

Based on the discoveries made by the inventors, speculations on mode of action for inhibitors binding at the NS5B allosteric site can be made. Because the binding of Inhibitor No. 1 and other compounds to the allosteric site does not appear to significantly alter the secondary/tertiary structure of the thumb domain (Fig. 5), nor alter interdomain interactions within the polymerase, its mode of inhibition remains unclear. Possibilties include:

(a) The presence of a compound bound in the allosteric "canyon" could perturb dynamic motions within the thumb domain necessary for normal enzymatic processing. For example, it has been proposed that a thumb domain beta-loop composed of strands βT3 (442-447) and βT4 (450-466) could interact with either the RNA template strand or the newly-synthesized RNA duplex (Lesburg et al. 1999; Ago et al. 1999; Bressanelli et al. 1999). Several "hinge" residues (the base of the loop) must rearrange in order for this loop to move, and these hinge residues are linked to alpha-helices which lie directly under the allosteric site. Thus dynamical restriction of the allosteric region via a bound inhibitor could indirectly affect the ability of the beta-loop to move freely. However, restriction of the protein's degrees of freedom by the inhibitor may be a much more complex and subtle process, e.g., restricting readjustments of inter-helical interactions within the thumb necessary for enzymatic activity.

(b) The bound compound could interfere with important protein/RNA interactions which occur on the outer thumb region, despite this region being distant from the central active site. The template RNA strand which feeds into the active site, or newly synthesized duplex RNA which is emerging from the active site, may fold back onto the thumb region and interact with the group of conserved basic residues found there (see Fig. 6); such an interaction could be perturbed when a small molecule is bound in the nearby "allosteric" canyon.

Relevance of the BK-NS5B allosteric site to other genotypes of HCV was also determined by the inventors. The sequence of amino acids comprising the definition of NS5B allosteric binding site (BK strain) is shown in Fig. 7 (a). Of these, about 50% are invariant among all HCV polymerase sequences. This number becomes 66% if conservation of amino acid type is considered for HCV (and HGV) sequences, where "type" implies physical character of the amino acid (positive charge, negative charge, small hydrophobic, etc.). An examination of the surface of the allosteric binding site, especially around the bound inhibitor, shows that it is composed largely of invariant or highly conserved residues (Fig. 7b). Therefore this site should retain a high degree of three-dimensional similarity across all HCV serotypes of NS5B polymerase, and thus remain complementary to inhibitors optimized for the BK strain. In other words, inhibitors developed for the BK enzyme should effectively target other serotypes of HCV and even HGV.

The following examples are provided to illustrate and further define the invention, and should not be construed as limiting the scope of the invention.

EXAMPLES

Example 1: The Mutated NS5B RNA polymerase

A. Cloning

The complete protein sequence for the native HCV NS5B RNA polymerase is shown as SEQ ID No. 1 (aa 1-591 of SEQ ID No.1). The cDNA sequence for the HCV NS5B protein, with 21 the amino acid C-terminal deletion, is amplified by PCR from a cDNA clone of the HCV-BK genome using the following primers:

forward primer:

GGAATTCCATATGTCAATGTCCTACACATGGACTGGCG (SEQ ID No. 9)

reverse primer:

CTGCTCGAGTCAGTGGTGGGTGGTGGTGGTGGCGAGGTCGGGCACGAGGAC

(SEQ ID No. 10)

Restriction sites NdeI and XhoI were added to aid cloning. The sites and codons for a (His)6 tag, and the stop codon were engineered in the PCR primers. Codons for the (His)6 tag preceded the stop codon, so that an expressed protein would have the (His)6 tag at its C-terminus.

The amplified cDNA was digested with NdeI and XhoI (Roche Molecular Biochemicals, Berkeley, CA) and cloned into expression plasmid pSW504 engineered at Agouron Pharmaceuticals, San Diego, California, and transformed into an *E.coli* strain DH5α (Invitrogen Life Technologies, Carlsbad, CA) according to vender's instructions. Plasmid DNA was isolated from the *E.coli* cells by standard methods (Qiagen, Venlo, Netherlands), and the sequences were verified by sequencing. (Retrogen, San Diego, CA and San Diego State University Core Facility, San Diego, CA).

Crystallzation of the wild type HCV-BK NS5B protein with C-terminal 21 amino acid deletion and $(His)_6$ tag revealed that the protein was crystallizable but crystal quality was insufficient to solve the structure.. In order to improve crystal quality, mutagenesis of the protein sequence was designed. Based on primary (Protean) and secondary structure (PHD) prediction (B. Rost, "PHD: predicting one dimensional protein structure by profile based neural networks," Methods in Enzymology, 266, 525-539, 1996) analysis, protein homology modeling (SEGMOD, Stanford University, Palo Alto, CA) and proteolysis patterns, two single point mutations MUT-1 and Mut-2 were chosen to improve crystallizability of the protein. Based on three-dimensional X-ray structure information of the MUT-1 and MUT-2 mutant (Agouron Pharmaceuticals, La Jolla, California), MUT-3 and MUT-4 were chosen to improve protein stability and solubility. Co-crystallization with compounds was done with the protein containing MUT-2, MUT-3, and MUT-4 mutations ("MUT-5").

Site-directed mutagenesis was performed using this plasmid as a template and standard mutagenesis techniques (Quickchange, Strategene, La Jolla, CA). To effect the site changes, the following oligonucleotides were used in successive rounds of PCR and restriction enzyme digestion:

For MUT-1: GGCTATGGGGCACAGGACGTCCGGAAC

(SEQ ID No. 11)

GTTCCGGACGTCCTGTGCCCCATAGCC

(SEQ ID No. 12)

For MUT-3: CATCTCGCAGCGCAGGCCAGCGGCAGAAGAAGGTC
(SEQ ID No. 13)
GACCTTCTTCTGCCGCTGGCCTGCGCTGCGAGATG
(SEQ ID No. 14)

For MUT-4: CGGCCAAAGCCAAGTACGGCTATGGGGCAAAG
(SEQ ID No. 15)
CTTTGCCCCATAGCCCGTACTTGGATTTGGCCG
(SEQ ID No.16)

For MUT-2: GGAACCTATCCAGCAGGGCCGTTAACCAC
(SEQ ID No. 17)
GTGGTTAACGGCCCTGCTGGATAGGTTCC
(SEQ ID No. 18)

After each reaction, the mutated DNA was transformed into *E.coli* cells (DH5α, Invitrogen Life Technologies, Carlsbad, CA). Plasmid DNA was isolated from these cells. The presence of desired mutations were confirmed by sequencing. After sequence confirmation of the existence of the desired mutation, the plasmid was subjected to the next round of mutagenesis. The full NS5B cDNA was sequenced to confirm no other mutations were introduced during PCR. The plasmids containing the mutants with the mutations MUT-1, MUT-2, MUT-3/MUT-2, MUT-4/MUT-2, and MUT-3/MUT-4/MUT-2 ("MUT-5") were used for protein expression. Only the proteins containing 1 or 3 mutations were used for crystallization.

B. Expression

Expression of the NS5B mutant fusion RNA polymerase protein was accomplished in DH5α cells (Invitrogen Life Technologies, Carlsbad, CA). Cells were grown to O.D. 600- 0.6 at 37°C. Expression was induced by the addition of 1μM isopropyl β-D-thiogalactopyranoside (IPTG) to the culture. Cells were incubated with IPTG for 12 hours at 30°C, and pelleted by centrifugation.

C. Purification

Purification of the RNA polymerase mutant was accomplished as follows. Cell pellets were resuspended and microfluidized (Microfluidizer, Microfluidics, Newton, MA) in lysis buffer (20 mM Tris, pH 8.0, 5 mM $MgCl_2$, 300 mM NaCl, 10% Glycerol, 12 mM 2-Mercaptoethenol, 1x complete EDTA-free protease inhibitor tablets, Roche Molecular Biochemicals, Berkeley, CA) at 4°C, followed by ultracentrifugation (Beckman Coulter, Brea, CA). The supernatant was removed and incubated with S7 nuclease (30units/μl) at 4°C for 30 minutes (Roche Molecular Biochemicals, Berkeley, CA). The supernatant was loaded on a SuperFlow Ni-nitrilotriacetic acid affinity column (Qiagen, Venlo, Netherlands), and the HCV NS5B polymerase was eluted with an imidazole gradient (60 mM to 250mM). The protein was further purified by using SP Fast Flow ion-exchange chromatography (Amersham Pharmacia Biotechnology, Piscataway, NJ) through a salt gradient (300mM to 1 M NaCl), followed by S-100 gel filtration chromatography (Amersham Pharmacia Biotechnology, Piscataway, NJ). The eluted protein was in a buffer containing 10 mM Hepes, pH 7.5, 400 mM NaCl, 2 mM TCEP, and concentrated up to 30 mg/ml by using Amicon stir cells. The protein was quickly frozen by liquid-$N_2$ and stored in -80°C prior to use.

Example 2: Preparation of Compound **1**, 3-(4-Amino-2-*tert*-butyl-5-methyl-phenylsulfanyl)-6-cyclopentyl-4-hydroxy-6-[2-(4-hydroxyphenyl)ethyl]-5,6-dihydro-pyran-2-one (Inhibitor No. 1)

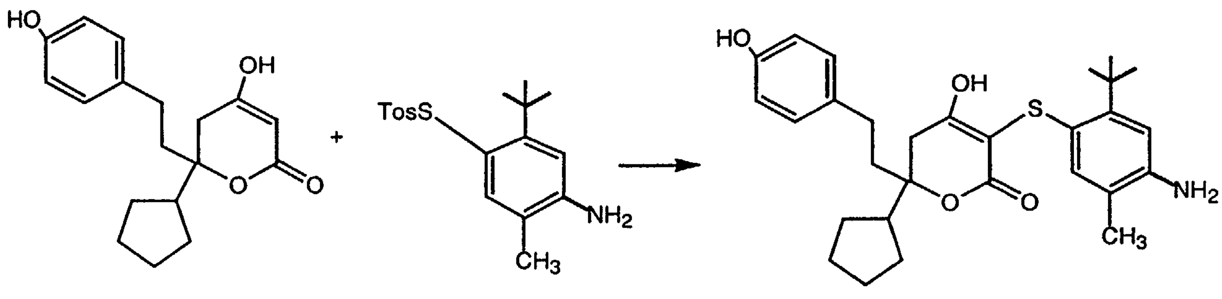

**A1** **B1** **1**

The title compound was synthesized by coupling intermediate 6-cyclopentyl-4-hydroxy-6-[2-(4-hydroxyphenyl)ethyl]-5,6-dihydro-pyran-2-one (**A1**) (prepared as described in: Vara Prasad, J. V. N., et al. *Bioorg. Med. Chem.* 2000, ***7***, 2775-2800) with intermediate toluene-4-thiosulfonic acid S-(4-amino-2-*tert*-butyl-5-methylphenyl) ester [**B1**, Tos = (4-$CH_3$)$PhSO_2$-] (or the corresponding hydrochloride salt) (prepared as described in: Boyer, F. E., et al. *J. Med. Chem.* 2000, ***43*,** 843-858) using methods analogous to those described in the above two references for the preparation of related 5,6-dihydropyran-2-ones. $^1$H NMR ($CDCl_3$, 300 MHz) $\delta$ 1.28-1.85 (m, 8H), 1.54 (s, 9H), 1.83 (s, 3H), 2.02-2.09 (m, 2H), 2.40-2.46 (m, 1H), 2.61-2.67 (m, 2H), 2.73 (d, 1H, $J$ = 18.0 Hz), 2.93 (d, 1H, $J$ = 18.0 Hz), 6.68-6.73 (m, 4H), 6.95-6.98 (m, 2H).

Example 3: Crystallization of HCV NS5B RNA polymerase single mutant constructs.

The truncated wild-type HCV NS5B RNA polymerase had proven to be very difficult to crystallize until the site-directed mutagenesis and generation of mutants by the inventors. Crystals of the MUT-2 mutant were used for the original determination of the enzyme's x-ray structure (i.e., apo form, without bound inhibitor). Crystals were grown at 21°C using the hanging-drop vapor diffusion method (Methods In Enzymology, Vol. 114, Section 2, 1985). A 2 microliter (µl) volume of protein solution (12 mg/ml) was mixed with an equal volume of reservoir solution consisting of 24% PEG 8000 (w/v), 0.175M ammonium sulfate, buffered to pH4.0 via acetic acid, and also containing 5mM benzamidine HCl. The crystals belong to space group P2(1)2(1)2(1), with unit cell 86, 106, 126 Å, and with two molecules per asymmetric unit. The solvent content of the crystals is about 45%. The crystals are thin plates, with maximum dimension about 0.2mm. Selenomethionine version of the MUT-2 mutant could also be crystallized under these conditions, giving the same crystal form, although the crystals were smaller. Another single mutant construct, MUT-1, was crystallized under conditions similar to MUT-2. The MUT-1 crystals had the same properties as the MUT-2 crystals (space group, unit cell, etc.).

Example 4: Crystal structure determination of HCV NS5B RNA polymerase mutant MUT-2.

Because the MUT-2 polymerase crystals grew at pH4.0, it was necessary to transfer them to higher pH buffers prior to exposure to the heavy metal compounds used for derivative screening. Most useful was a MES pH6.5 buffer containing 27% PEG 8000. Crystals were transferred into these solutions via small loops, and soaked for about 24 hrs. Diffraction data were initially collected in-house, on a Rigaku rotating anode generator equipped with a Mar345 image plate detector. Data were processed with Denzo/Scalepack (Otwinowski and Minor, 1997). All data were collected at -170°C: crystals were flash-cooled in liquid nitrogen, after a quick emersion into a 20% glycerol cryoprotectant.

No evidence for binding of heavy atoms (from anomalous Patterson analysis) was seen using a variety of platinum, mercury, lead, and uranyl compounds. However, an anomalous signal was detected for lanthanides samarium ($Sm^{3+}$) and erbium chloride, and conditions were eventually refined to 1-2 mM $SmCl_3$. A determination of the samarium positions was made which explained the anomalous Patterson peaks; there is one $Sm^{3+}$ per molecule at a similar position (thus two ions per asymmetric unit dimer). These same crystals were stored frozen and later transported to a synchrotron x-ray source for further analysis using the multiwavelength anomalous dispersion (MAD) method (see

below). Several small MUT-2 selenomethionine crystals were grown, and one had suitable diffraction for a MAD experiment at a synchrotron source.

The polymerase crystals described above were used for x-ray data collection at the Advance Photon Source (APS) in Argonne National Laboratory (Beamline 17ID). These include two samarium-soaked MUT-2 crystals and one MUT-2 selenomethionine crystal. Each crystal was used to collect three tuned-wavelength data sets, where the x-ray wavelength was tuned to be at/near the electronic absorption peak of the target atom (either Sm3+ or selenium). Typical exposure time was 3-5 seconds per 1.0 degree oscillation. The detector was a MarCCD 165. The wavelengths were tuned to either peak (max. f"), edge (max. f'), or high remote (peak+100ev). For samarium the wavelengths are around 1.84Å; for selenomethionine (Semet) they are about 0.98Å. Samarium data (at any wavelength) was complete to the detector edge of 2.56Å, with Rsym~10%, and overall redundancy of 4.9 (after anomalous scaling). The one selenomethionine crystal yielded three tuned-wavelength data sets, each complete to 2.1Å, with Rsym~9%, and redundancy of 7.1. Mosaicity for all crystals was about 0.50.

After in-house processing of the APS results, the samarium data showed a strong anomalous signal at either the peak or edge wavelengths, as seen by anomalous Patterson maps. Difference Patterson maps between different wavelength amplitudes revealed a strong dispersive signal for edge-to-remote and edge-to-peak. The isomorphous and anomalous differences were combined, along with the known samarium positions, and used for phase refinement & solvent-flattening in PHASES (Furey and Swaninathan, 1990). A density map with well-defined boundaries and elements of secondary structure emerged, despite only one Sm3+ present per 65 kilodalton protein. The samarium site lies at the center of the molecule, as expected if it binds at the enzyme's magnesium site.

For the selenomethionine data, prominent peaks appeared in anomalous Pattersons at peak and edge wavelengths, but there was little dispersive signal, as shown by featureless Pattersons between wavelengths. Nevertheless, the anomalous portion of this data (Bijvoet pairs from peak and edge data) was combined with the samarium information. To locate the selenium sites in the asymmetric unit (22 theoretically), phases from the pure-samarium case were used to generate a Bijvoet-difference Fourier for the selenomethionine peak data. The top 22 (unique) peaks in this Fourier were significantly higher than the rest, and assumed to be the seleniums. The statistics from PHASES (before solvent-flattening) for the combination of samarium (2-site) + selenomet. (22-site) at 2.7Å were: R(Cullis) for Sm SIR = 64%; Overall F.O.M = 0.756; Overall Phasing Power = 1.1 for Sm SIR, 2.3 for Sm SAS, and 3.2 for selenomethionine. SAS. After solvent-flattening and non-crystallographic averaging over the two copies, a 2.7Å density map was generated which showed good connectivity at 1.5 sigma over most of the molecule. Many sidechains could be identified by their shape, and the methionine positions verified by superposition of the cross-Fourier selenium peaks.

A nearly-complete model was built into this map, using XFTT (McRee, 1992). The model was then transferred to high resolution data that had been obtained from MUT-2 crystals (to 2.0 Å) at the Stanford Synchrotron Radiation Laboratory (SSRL), Palo Alto, CA, and refined with XPLOR (Brunger, 1992). MUT-1 crystals were used to collect even higher resolution data (1.76Å) at SSRL, and this data allowed further refinement of the NS5B model. Our current model of single-mutant apo NS5B consists of residues 1-148, 154-533, 535-563 in molecule 1, and residues 1-148, 154-533, 539-563 in molecule 2 (2 molecules per asymmetric unit), with a total of five sulfate groups and 526 water molecules. The R-factor is 24% at 1.76Å.

Example 5: Description of the structure of HCV NS5B RNA polymerase.

HCV NS5B RNA polymerase has a three domain organization, conventionally labeled "fingers", "palm", and "thumb". There are extensive contacts between the fingers and thumb which leads to an overall globular shape, distinct from the U-shape found in most other polymerases. The HCV polypeptide chain completely encircles the active site deep within its center, this site containing the amino acids required for RNA synthesis.

The thumb domain of NS5B, which includes residues 371-563, consists of seven alpha-helices and two double-stranded anti-parallel beta-loops. This domain has the general appearance of a bundle of helices wrapped by two beta loops. The alpha helices are defined here as αT1 (residues 389-400), αT2 (407-415), αT3 (419-437), αT4 (459-466), αT5 (480-492), αT6 (498-513), and αT7 (517-524). One beta loop is defined by strands βT1 (368-375) and βT2 (378-386); another beta loop by strands βT3 (442-447) and βT4 (450-466). These definitions are refered to later in the description of the "allosteric site" for bound inhibitors.

The amino acid content of the thumb region is unusally rich with arginines and lysines. For example, of the 13 residues between positions 498 and 510, six are arginines residing on a single alpha-helix. Mutagenesis experiments have suggested a functional role for arginines in the 500-505 segment (Yamashita et al., 1998). The spatial arrangement of basic sidechains in the thumb domain gives the appearance of a continuous strip of positive charge spanning the thumb's surface (Fig. 6), suggesting a role in the binding of negatively-charged olignonucleotide. The proximity of this charge to the inhibitor binding site is discussed later.

Example 6: Cocrystallization of NS5B RNA polymerase triple-mutant complexed with compound.

The crystal structure of MUT-2 NS5B polymerase revealed two solvent-exposed, hydrophobic residues, Phe 101 and Leu 47, which became candidates for mutagenesis into more polar amino acids, as found at these positions in other HCV strains. This led to the preparation of a triple mutant construct (MUT-2/MUT-4/MUT-3 or MUT-5), in order to create a more soluble protein. Initial experiments aimed at obtaining a cocrystal structure involved incubation of the triple-mutant construct with a low micromolar inhibitor, followed by the hanging-drop vapor-diffusion method. The protein/inhibitor solution was mixed with precipitating reagents in equal volumes. This mixture was dispensed onto a glass coverslip (about 6 microliters total), inverted, and sealed over a reservoir containing the crystallization reagents. Buffer conditions under which the crystals were grown included: (1) 0.2M ammonium sulfate, 0.1M ammonium acetate, 30% monomethyl polyethylene glycol 2000, and pH 4.6; or (2) 2.0M ammonium sulfate only, at pH 5.0; or (3) 1.2-1.8M potassium phosphate, 100mM sodium citrate pH 5.5. The crystals belong to space group P4(1)2(1)2, with unit cell of 83,83,180 Å, and contain one molecule per asymmetric unit.

Example 7: Crystal structure determination of HCV NS5B polymerase-inhibitor complexes.

The crystals grown in the above example were used to collect x-ray diffraction data to 2.2 Å resolution. The data was collected in-house as described above for the single-mutant crystals. The previously-solved in-house structures of the single-mutant MUT-1 and MUT-2 HCV polymerase (see above) were used to initiate the computer technique of Molecular Replacement using the software AmoRe (Navaza, 1994). This allowed determination of the triple-mutant polymerase structure within these polymerase-inhibitor crystals. The atomic coordinates of the triple-mutant enzyme were then refined by computer using XPLOR. The bound inhibitor was identified as described in the next section. Further refinement was done with ARP/wARP (Perrakis et al., 1999), which allowed identification of missing loops. The enzyme model within the complex now consists of residues 1-563. The final R-factors are 19.6% using XPLOR and 17.3% using ARP/wARP, both at 2.22 Å resolution. Figs. 1 and 2 are overall views of the enzyme with bound Inhibitor No. 1.

The three-dimensional structures for inhibitor-bound NS5B and unbound NS5B are very similar. Superposition of the apo-structure onto the enzyme/inhibitor complex reveals only minor rearrangement of residues in the thumb domain surrounding the bound inhibitor (Fig. 5a). The thumb domains from unbound and bound enzymes can be superimposed with an rms difference of only 0.29 Å on alpha-carbons. Closer inspection reveals that residues 495-499, forming a loop just prior to alpha helix $\alpha$T6, shift about 1 Å to accommodate the inhibitor. Also, the sidechains of residues Leu 497, Met 423, and Leu 419 rearrange upon inhibitor binding; this rearrangment involves alternate rotomer positions (about torsion angle $\chi_1$), see Fig. 5b.

Example 8: Identification of the HCV NS5B polymerase allosteric binding site.

Once the structure of the triple-mutant NS5B polymerase from the cocrystals had been solved and refined, electron density maps were examined to search for "extra" density not accounted for by the protein. In a cleft on the outer perimeter of the thumb domain of the enzyme, density was observed which could be interpreted as a bound small molecule, namely the compound that had been included in the cocrystallization, Inhibitor No. 1. A model for the bound Inhibitor No. 1 was built and refined along with the protein. See Figs. 1-3.

Subsequently, other cocrystal structures were solved involving compounds that are closely related to the original described above. These compounds all bound similarly, in the same cleft on the thumb domain of the enzyme. The nature of their interactions with the protein (shape complementarity, etc.) correlated well with their biochemical inhibition profiles. Thus it was established that the binding mode observed in the cocrystals reveals the manner in which these compounds act on the protein during biochemical inhibition assays. Because the compounds under discussion bind within a cleft on the polymerase "thumb" region far from the known active site, and because this binding can nevertheless inhibit the enzyme's function, this cleft can be defined as an "allosteric binding site". That is, the inhibitors operate indirectly (from large distances) to interfere with the polymerase's function.

Example 9: Assay of HCV NS5B RNA Polymerase and Inhibitors thereof.

Recombinant HCV polymerase was tested for its ability to perform primer/template-directed transcription in assays that contained 30mM tris-HCl pH 7.2, 10mM $MgCl_2$, 20mM NaCl, 1 mM Dithiothreitol (DTT), 0.05% Tween-20, 1% glycerol, 5 pmoles biotin-$dG_{12}$ (primer), 0.5 pmoles poly$(rC)_{300}$ (template), 1µM GTP, 0.1-0.3 µCi $\alpha$-$^{32}$P-GTP, and 2.5 pmoles (0.15µg) HCV polymerase protein in a final volume of 75µl. Reactions were initiated by addition of enzyme and incubated 30 minutes at 30°C. Reactions were stopped by addition of 33mM EDTA and polynucleotide products were collected by filtration through Diethylaminoethyl (DE) Filtermat papers (Wallac); unincorporated triphosphate was

removed by washing the filters with 5% dibasic sodium phoshate. The filters were counted in a Packard Tri-Lux Microbeta scintillation counter. Compounds to be tested were added at various concentrations from stocks in 10% DMSP-water (final DMSO=1% reaction). $IC_{50}$ values were estimated from the primary cpm data (collected in triplicate) using the formula:

$$Cpm(I) = cpm\ (no\ inhibitor)^* (1-[I]/([I] + IC_{50}))$$

Figure 8 describes the results of fitting the primary data to the above formula. Curve fitting was performed in the program KaleidaGraph.

Sequence Listing:

| | |
|---|---|
| SEQ ID No. 1 | Native RNA Polymerase |
| SEQ ID.No. 2 | DNA encoding the mutant RNA Polymerase |
| SEQ ID No. 3 | Mutant RNA Polymerase MUT-2 |
| SEQ ID No. 4 | Mutant RNA Polymerase MUT-1 |
| SEQ ID No. 5 | Mutant RNA Polymerase MUT-5 |
| SEQ ID No. 6 | DNA encoding Mutant RNA Polymerase MUT-2 |
| SEQ ID No. 7 | DNA encoding Mutant RNA Polymerase MUT-1 |
| SEQ ID No. 8 | DNA encoding Mutant RNA Polymerase MUT-5 |
| SEQ ID No. 9 -10 | Sequencing primers |
| SEQ ID No. 11-18 | PCR primers |

## Claims

1. An isolated, purified nucleic acid molecule, which encodes a mutant HCV NS5B RNA polymerase, wherein a point mutation occurs at least at one or all of positions 47, 101, 106, and/or 114 of SEQ ID No. 1.

2. The nucleic acid molecule of claim 1, further comprising the DNA sequence of SEQ ID No. 6, or a fragment thereof.

3. The nucleic acid molecule of claim 1, further comprising the DNA sequence of SEQ ID No. 7, or a fragment thereof.

4. The nucleic acid molecule of claim 1, further comprising the DNA sequence of SEQ ID No. 8, or a fragment thereof.

5. An isolated, purified nucleic acid molecule that encodes a mutant HCV NS5B RNA polymerase, wherein said mutant HCV NS5B RNA polymerase has improved crystallization properties as compared to a native HCV NS5B RNA polymerase.

6. A mutant HCV NS5B RNA polymerase comprising one or more amino acid point mutations, or a fragment thereof, wherein said amino acid point mutations are located at positions 47, 101, 106, and/or 114 of SEQ ID No. 1.

7. The mutant HCV NS5B RNA polymerase of claim 6, comprising SEQ ID No. 3.

8. The mutant HCV NS5B RNA polymerase of claim 6, comprising SEQ ID No. 4.

9. The mutant HCV NS5B RNA polymerase of claim 6, comprising SEQ ID No. 5.

10. A crystal structure of the mutant HCV NS5B RNA polymerase comprising one or more amino acid point mutations, or a fragment thereof, or an active analog thereof, wherein said amino acid point mutations are located at positions 47, 101, 106, and/or 114 of SEQ ID No. 1.

11. A crystal structure of the mutant HCV NS5B RNA polymerase comprising SEQ ID No. 3.

12. A crystal structure of the mutant HCV NS5B RNA polymerase comprising SEQ ID No. 4.

13. A crystal structure of the mutant HCV NS5B RNA polymerase comprising SEQ ID No. 5.

14. An expression vector for producing a mutant HCV NS5B RNA polymerase, which vector comprises a nucleic acid molecule encoding the mutant HCV NS5B RNA polymerase of claim 6, operably linked to expression regulatory control sequences.

15. A host cell stably transformed with a nucleic acid molecule encoding the mutant HCV NS5B RNA polymerase of claim 6 in a manner allowing the expression of said mutant HCV NS5B RNA polymerase.

16. An isolated, purified mutant nucleic acid molecule, wherein nucleic acids 418 to 540 encode an allosteric binding cleft comprising a region of the Hepatitis C virus (HCV) NS5B RNA polymerase, or a fragment thereof.

17. The nucleic acid molecule of claim 16, wherein said allosteric binding cleft comprises amino acids 460 to 540 and 418 to 435 of SEQ ID No. 1.

18. The nucleic acid molecule of claim 17, wherein said region from 418 to 435 is an alpha helix.

19. The nucleic acid molecule of claim 18, wherein said allosteric binding cleft is about 25Å long, 15Å wide and up to 10Å deep.

20. The nucleic acid molecule of claim 16, wherein said allosteric binding cleft is removed from the central active-site cavity employed in RNA strand synthesis.

21. The nucleic acid molecule of claim 20, wherein said allosteric binding cleft is located about 30Å from the central active site cavity employed in RNA strand synthesis.

**22.** An isolated polypeptide, wherein nucleic acids 418 to 540 encode an allosteric binding cleft comprising a region of the Hepatitis C virus (HCV) NS5B polymerase.

**23.** The polypeptide of claim 22 wherein said binding cleft comprises amino acids 460 to 540 and 418 to 435 of SEQ ID No. 1, or a conservatively substituted variant thereof.

**24.** The polypeptide of claim 23, wherein the region from 418 to 435 is an alpha helix.

**25.** The polypeptide of claim 22, wherein said allosteric binding cleft is about 26Å long, 15Å wide and up to 10Å deep.

**26.** An expression vector for producing an HCV NS5B RNA polymerase allosteric binding cleft, which vector comprises a nucleic acid molecule encoding the allosteric binding cleft, operably linked to expression regulatory control sequences.

**27.** The expression vector of claim 26 wherein said allosteric binding cleft comprises amino acids 460 to 540 and 418 to 435 of SEQ ID No. 1, or a conservatively substituted variant thereof.

**28.** The expression vector of claim 26 wherein said allosteric binding cleft is about 26A long, 15A wide and up to 10A deep.

**29.** A host cell stably transformed with a nucleic acid molecule encoding an allosteric binding cleft of HCV NS5B polymerase or analog thereof in a manner allowing the expression of said allosteric binding cleft.

**30.** A method for assaying for a candidate compound for its ability to interact with an HCV NS5B RNA polymerase allosteric binding cleft comprising:

(a) expressing a nucleic acid molecule which encodes an allosteric binding cleft comprising a region of the Hepatitis C virus (HCV) NS5B RNA polymerase in a host capable of producing said binding cleft;

(b) exposing said binding cleft to said candidate substance; and

(c) evaluating the interaction of said binding cleft.

**31.** A method of drug design for compounds which interact with the HCV NS5B RNA polymerase allosteric binding cleft comprising:

(a) crytallizing said allosteric binding cleft;

(b) obtaining data from the crystal structure using computer programs;

(b) applying the data generated from the crystal structure to a computer algorithm which generates a model of said allosteric binding cleft for use in designing molecules.

**32.** The method of claim 31 wherein the method is utilized to identify inhibitors of said polymerase, said inhibitors serving as lead compounds for the design of potentially therapeutic compounds for the treatment of HCV.

**33.** A method of using a computer processor for analyzing a molecular structure comprising:

a. a machine readable data storage medium comprising a data storage material encoded with machine readable data wherein said data comprises crystal coordinates of mutant HCV NS5B RNA polymerase,
b. a working memory for storing instructions for processing said machine readable data,
c. a CPU coupled to said working memory and said machine readable data for performing a Fourier transformation of the machine readable data and for processing such data into crystal coordinates, and
d. a display coupled to said CPU for displaying said crystal coordinates of said three-dimensional molecule or complex.

**34.** The method of claim 43 wherein the machine readable data storage medium is CD-ROM.

**35.** The method of 43 wherein the machine readable data storage medium is a magneto-optic disk.

**36.** A computer based method for processing X-ray coordinate data into three-dimensional graphical display of a HCV NS5B RNA polymerase molecule or molecular complex which comprises an allosteric binding domain.

**37.** The method of claim 46 wherein said X-ray coordinate data is stored in a machine readable storage medium.

**38.** The method of claim 46 wherein said three-dimensional graphical display is displayed on a computer monitor.

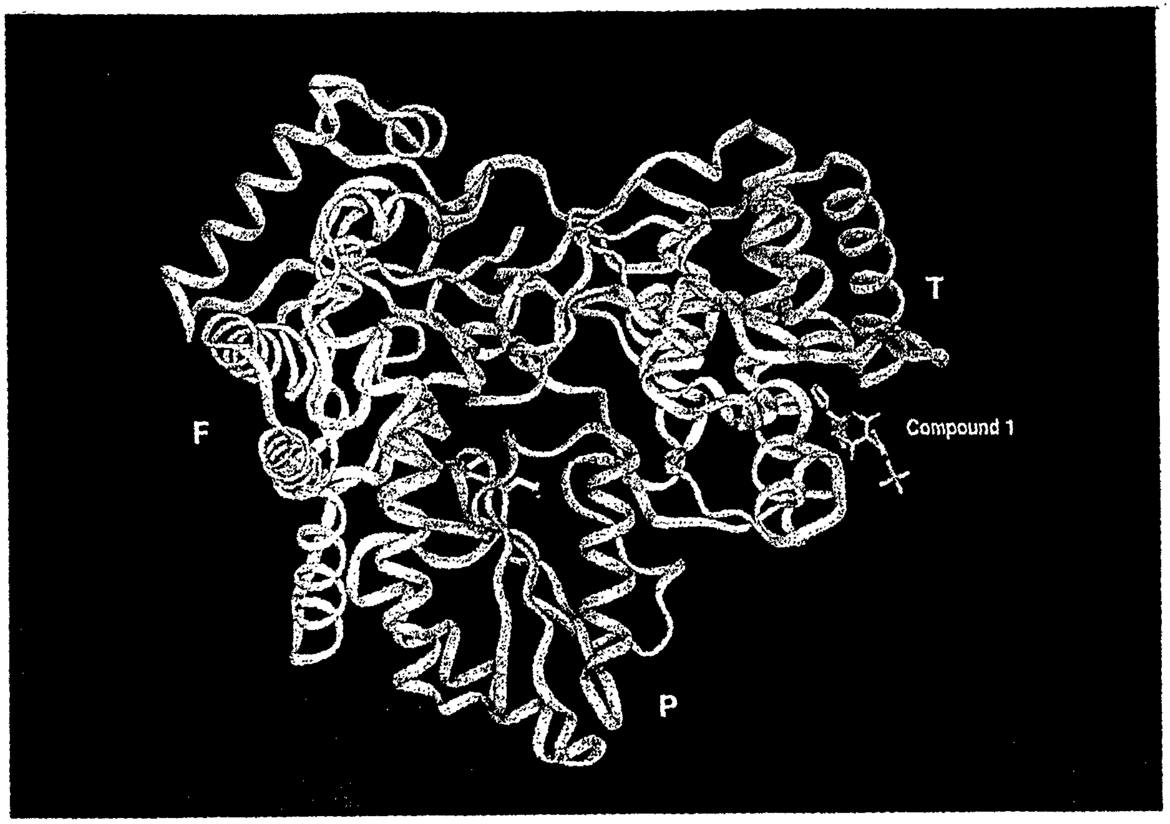


Fig. 1.

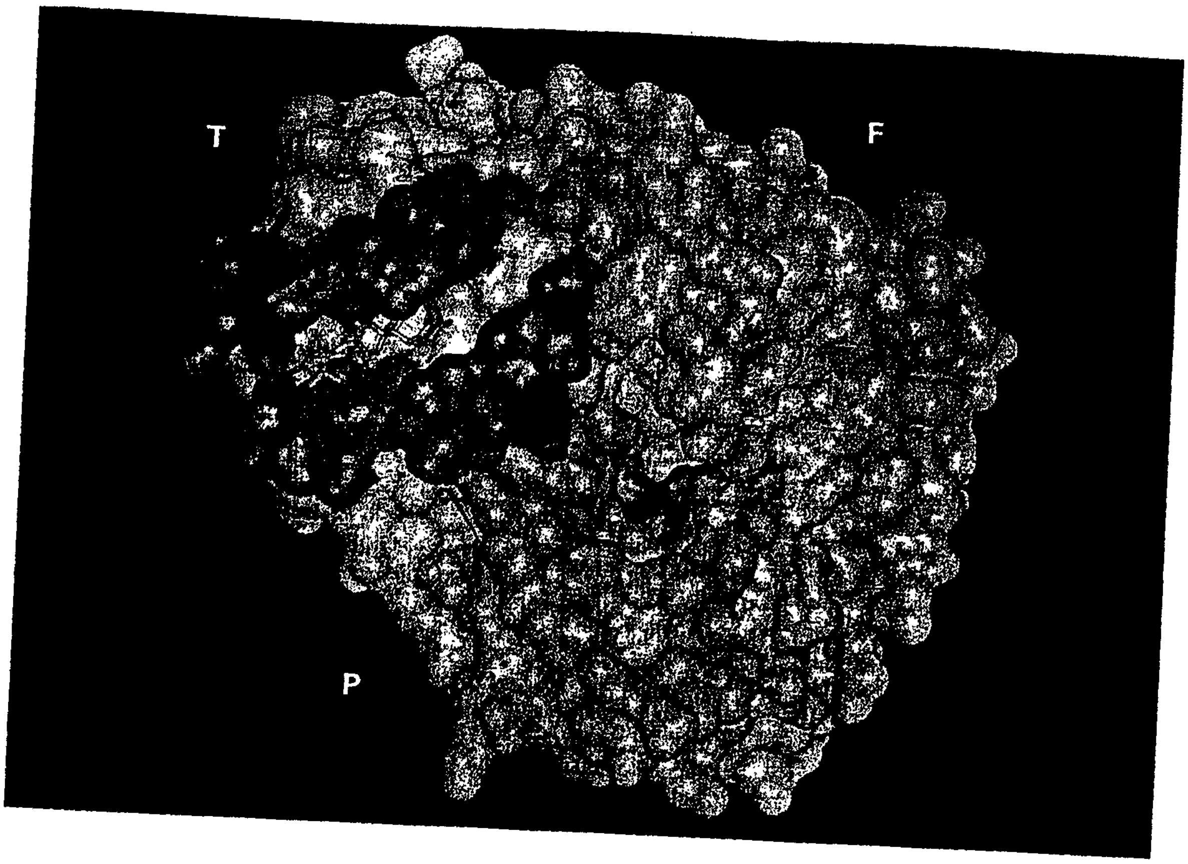


Fig. 2.

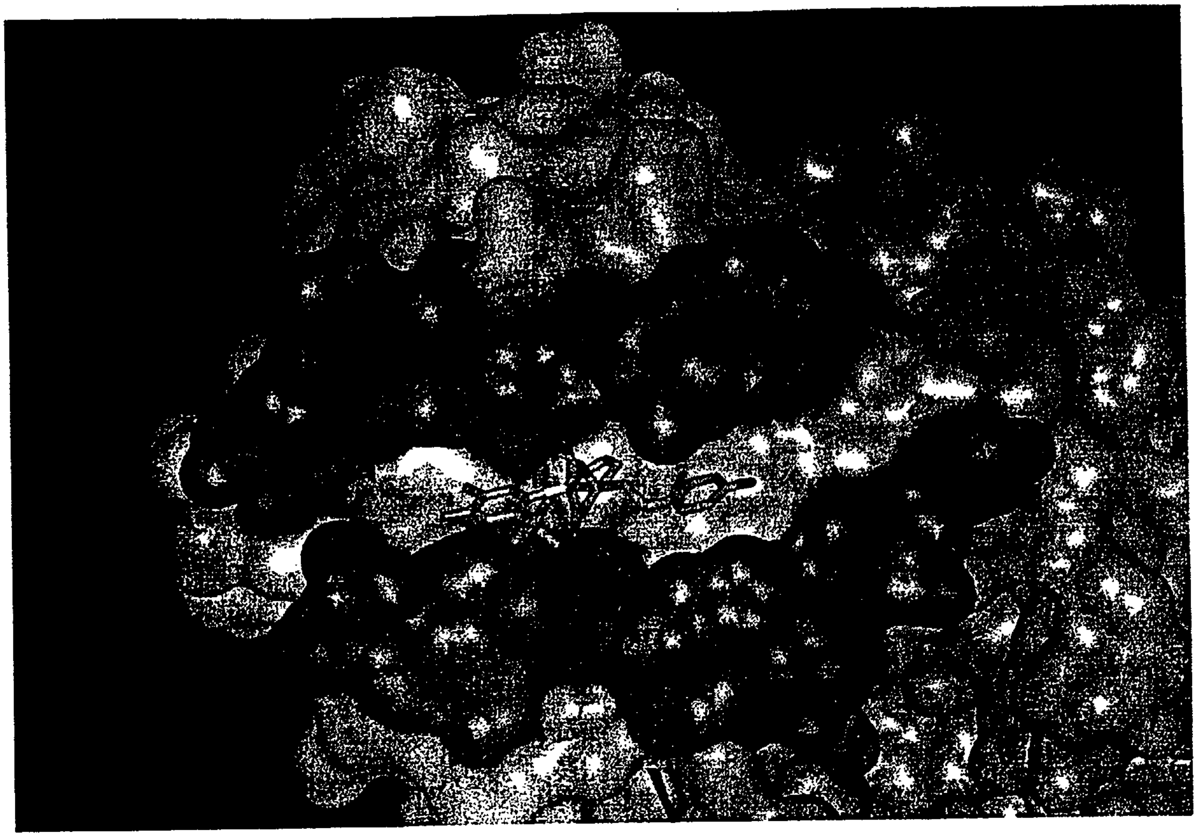

Fig. 3

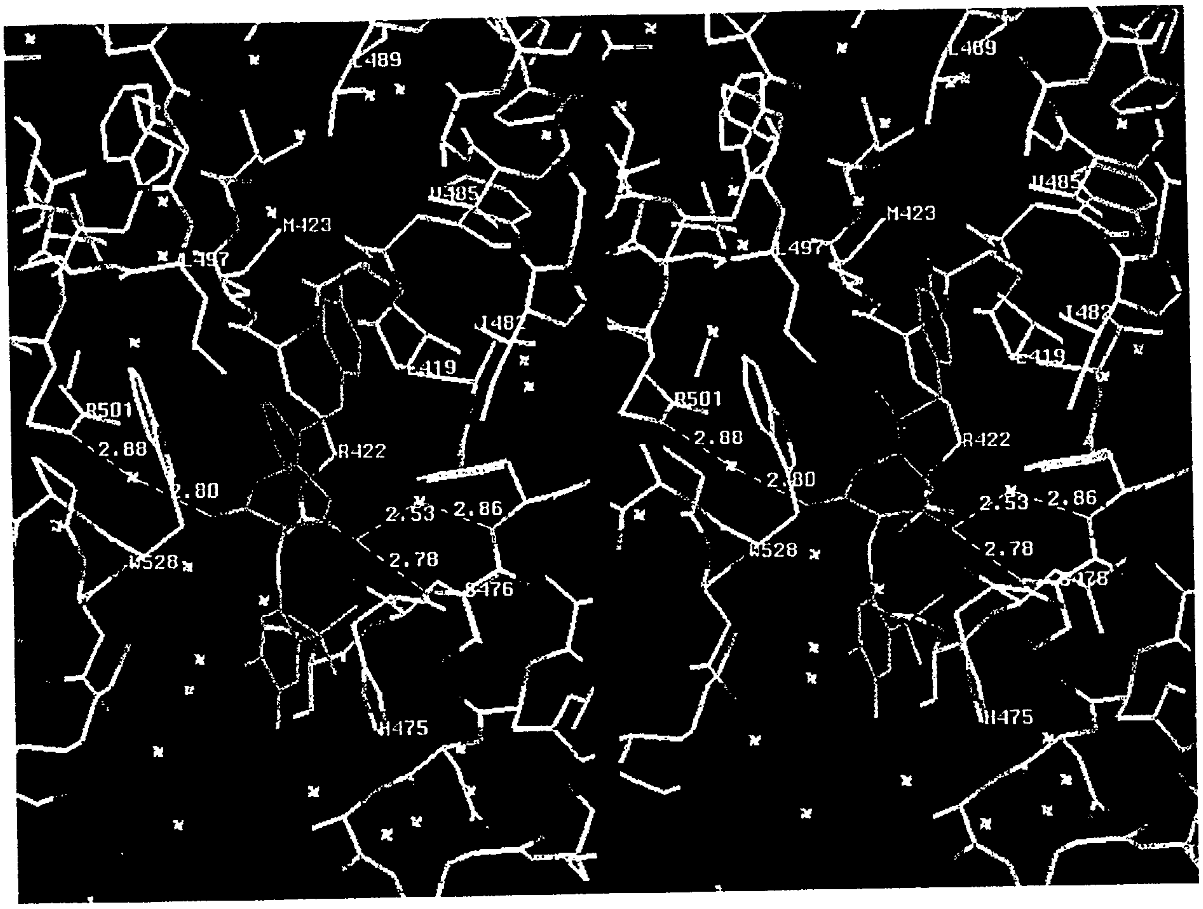
L489
H485
M423
L497
I482
L419
R501
2.88
2.80
R422
2.53
2.86
2.78
W528
S476
H475
L489
H485
M423
L497
I482
L419
R501
2.88
2.80
R422
2.53
2.86
2.78
W528
S476
H475

Fig. 4.

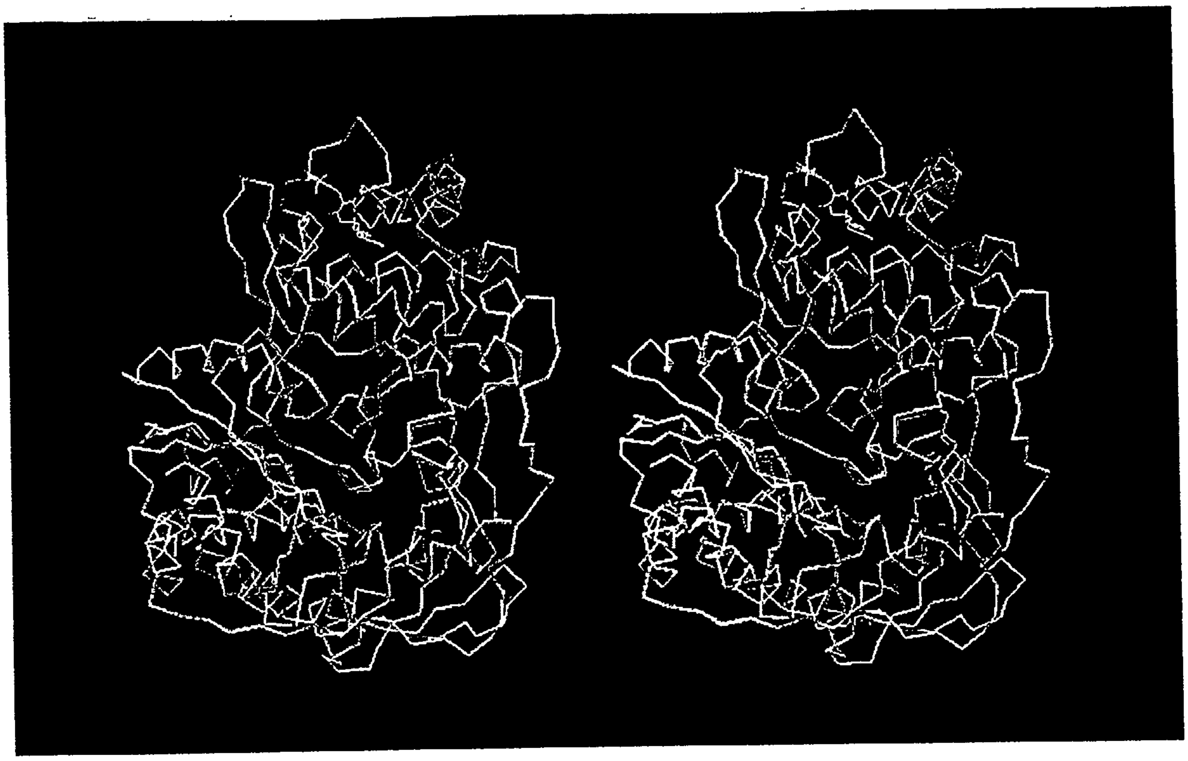

Fig. 5(a).

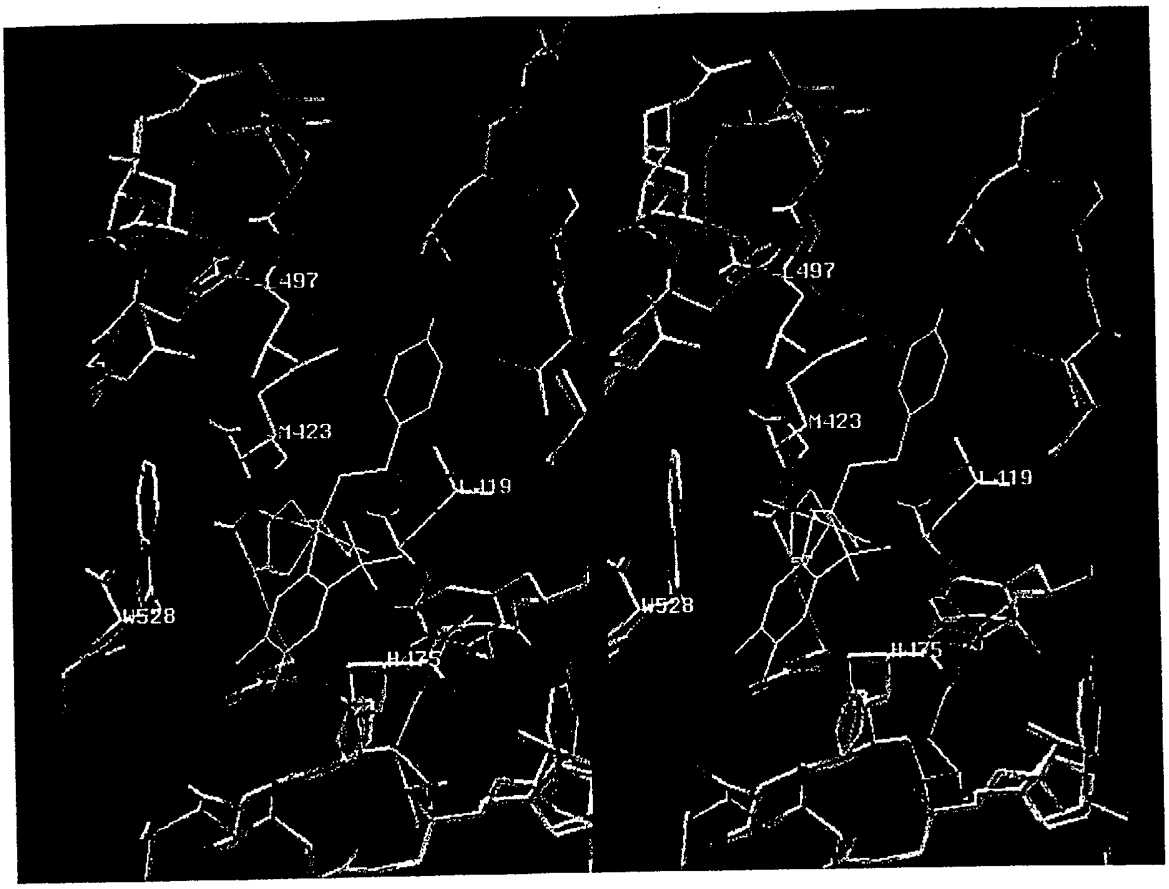


Fig. 5(b).

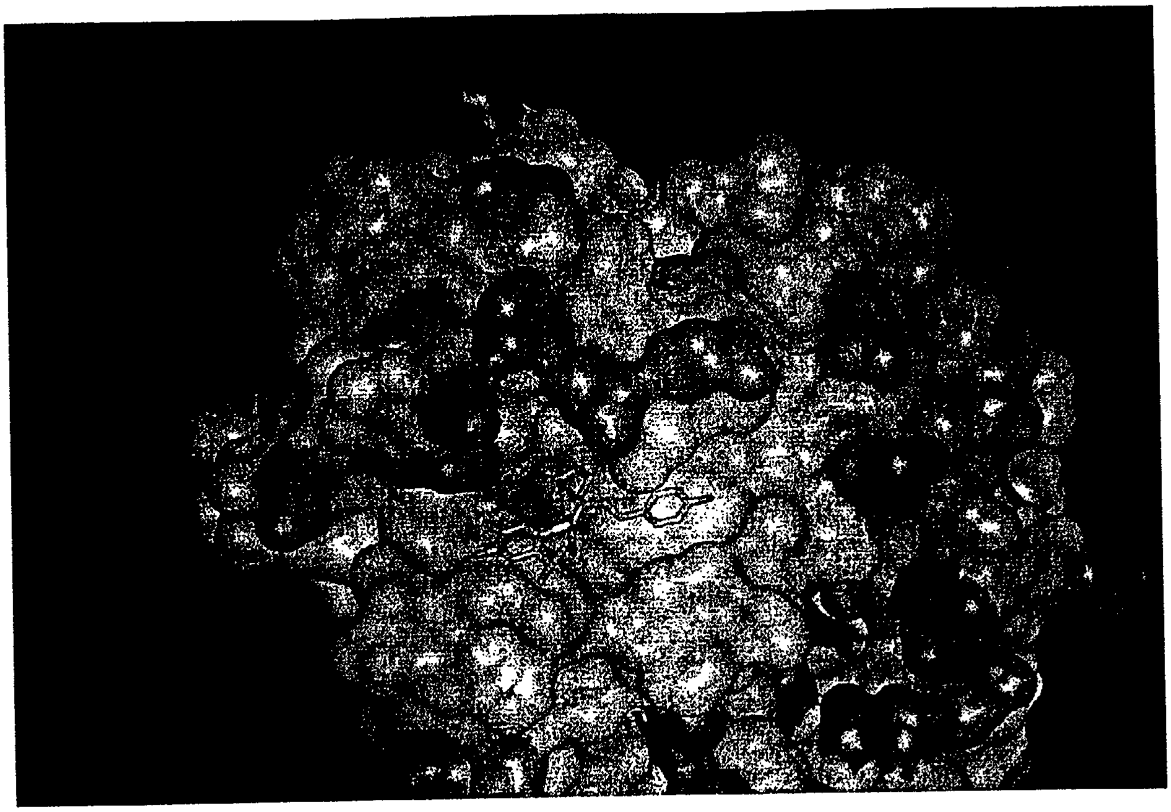

Fig. 6.

374 H *D* A S G 378 .... 416 A P T *L* W A R M *I* *L* *M* T 427

469 *L* S A F *S* L H S Y *S* P G E *I* *N* R V A S C L R 490

493 G V P P L R V W *R* 501

524 Y L F N W A *V* *K* T K L K L 536

Fig. 7(a).

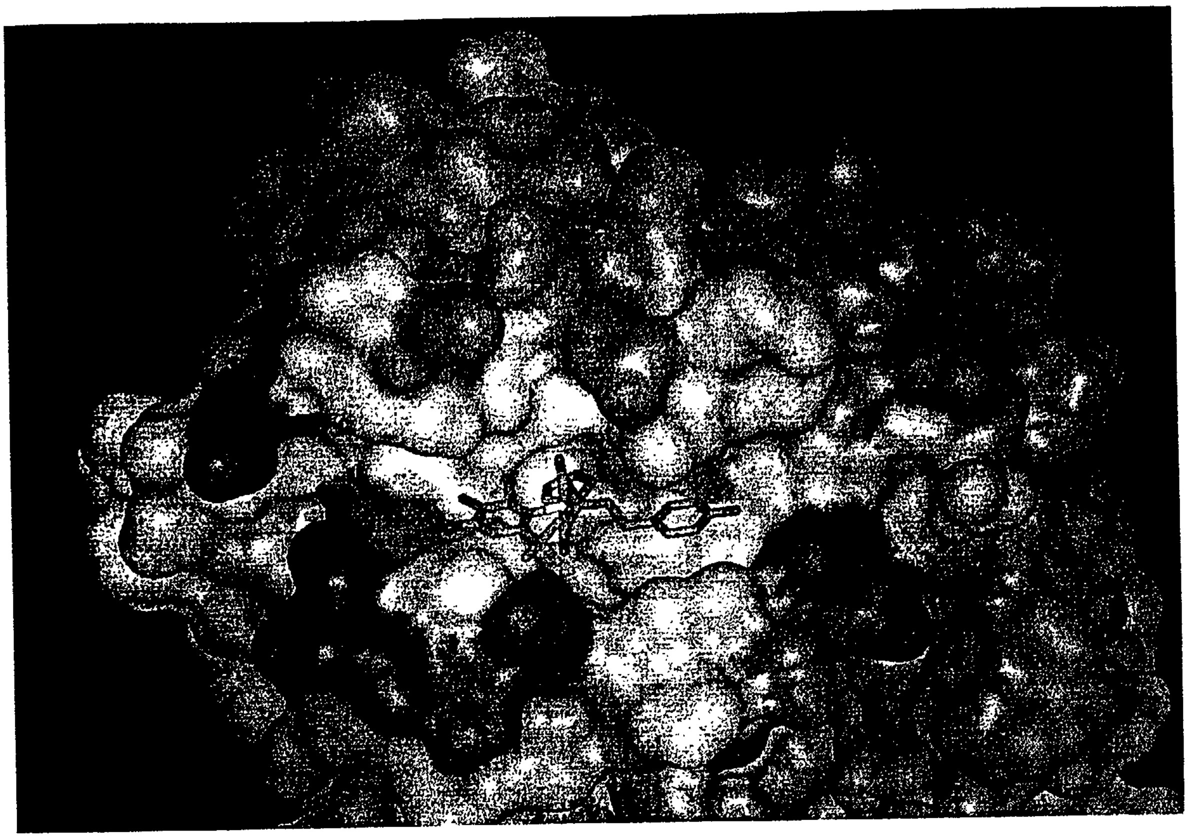

Fig. 7(b).

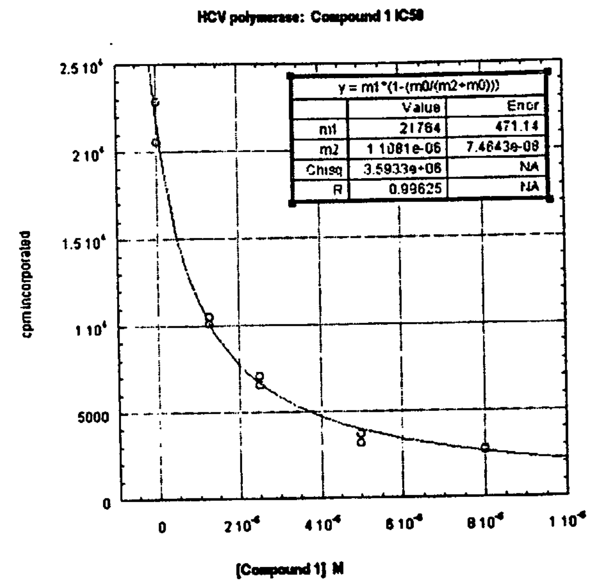
HCV polymerase: Compound 1 IC50
y = m1*(1-(m0/(m2+m0)))
Value
Error
m1
21764
471.14
m2
1.1081e-06
7.4643e-08
Chisq
3.5933e+06
NA
R
0.99625
NA
cpm incorporated
[Compound 1] M

Fig. 8